(19) **Europäisches Patentamt**
**European Patent Office**
**Office européen des brevets**

(11) **EP 4 151 263 A1**

(12) **EUROPEAN PATENT APPLICATION**
published in accordance with Art. 153(4) EPC

(43) Date of publication:
**22.03.2023 Bulletin 2023/12**

(21) Application number: **20942379.7**

(22) Date of filing: **22.06.2020**

(51) International Patent Classification (IPC):
**A61M 21/02** (2006.01)

(52) Cooperative Patent Classification (CPC):
(C-Sets available)
**A61M 21/02; G16H 20/70; G16H 40/63;**
**G16H 50/20; G16H 50/30; G16H 50/70;**
A61B 5/374; A61B 5/4812; A61B 5/4815;
A61M 2021/0027; A61M 2205/3553;
A61M 2205/3584; A61M 2205/3592;
A61M 2205/505; A61M 2205/84; (Cont.)

(86) International application number:
**PCT/CN2020/097389**

(87) International publication number:
**WO 2021/258245 (30.12.2021 Gazette 2021/52)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **HUAWEI TECHNOLOGIES CO., LTD.**
**Shenzhen, Guangdong 518129 (CN)**

(72) Inventors:
• **YANG, Hui**
**Shenzhen, Guangdong 518129 (CN)**

• **LIU, Chang**
**Shenzhen, Guangdong 518129 (CN)**
• **ZHANG, Huimin**
**Shenzhen, Guangdong 518129 (CN)**
• **LI, Xu**
**Shenzhen, Guangdong 518129 (CN)**

(74) Representative: **Pfenning, Meinig & Partner mbB**
**Patent- und Rechtsanwälte**
**Theresienhöhe 11a**
**80339 München (DE)**

(54) **METHOD AND DEVICE FOR UPDATING SLEEP AID AUDIO SIGNAL**

(57) A sleep-aiding audio signal updating method and apparatus in the artificial intelligence (AI) field is provided. The method includes: obtaining a first biological signal collected when a first audio signal in a sleep-aiding audio library is played (S510), where the first biological signal is a biological signal of a first user; determining a sleep quality of the first user based on the first biological signal (S520); and updating the sleep-aiding audio library based on the sleep quality of the first user (S530), so that a sleep-aiding audio signal can be updated, to provide a proper sleep-aiding audio signal for a user, and ensure a sleep-aiding effect.

FIG. 5

**(Cont. next page)**

(52) Cooperative Patent Classification (CPC): (Cont.)
A61M 2230/04; A61M 2230/10; A61M 2230/14;
A61M 2230/16

C-Sets
A61M 2230/04, A61M 2230/005;
A61M 2230/10, A61M 2230/005;
A61M 2230/14, A61M 2230/005;
A61M 2230/16, A61M 2230/005

**Description**

**TECHNICAL FIELD**

Embodiments of this application relate to the field of artificial intelligence, and in particular, to a sleep-aiding audio signal updating method and apparatus.

**BACKGROUND**

Sleep plays an important role in human life. With the development of society, the change of life and work habits due to modernization, and increasing pressure, an insomnia problem is becoming more serious, and a human sleep quality is also becoming lower. Insomnia may lead to a decline in memory, a decline in academic performance, inefficiency of work, and a decline in a life quality, and even lead to a threat to physical health and life safety. Therefore, it is increasingly important to study a method for effectively treating insomnia.

Main treatment methods in the industry include psychotherapy, drug therapy, food therapy, physiotherapy, and the like. The psychotherapy has an unstable curative effect and is affected by many factors, and can play only an auxiliary treatment role. The drug therapy has a relatively good curative effect, but long-term drug use is prone to cause tolerance, dependence, and withdrawal reactions, and side effects. Compared with conventional therapy, emerging physiotherapy, such as sound therapy, micro-current stimulation therapy, or electromagnetic stimulation-induced sleep therapy, has advantages such as safety and small side effects. Therefore, the study of sleep physiotherapy has become a main direction in current sleep disorder treatment. Because of advantages such as easy implementation and low costs, the sound therapy has become a method that receives wide attention in the physiotherapy.

Currently, various music sleep-aiding applications (APP) exist on the market, and can help users sleep. This type of APP usually includes a plurality of modules, such as night rain for sleep, brain wave for sleep, afternoon nap, vigor morning, and meditation. Generally, a user selects a corresponding module based on a need of the user, and then plays a sleep-aiding audio signal in the module. However, the sleep-aiding audio signal in the module is usually set by a system by default or is determined based on a user selection status. The sleep-aiding audio signal does not necessarily play a good sleep-aiding role for the user, that is, a sleep-aiding effect cannot be ensured.

Therefore, how to select a suitable sleep-aiding audio signal becomes a problem to be urgently resolved.

**SUMMARY**

This application provides a sleep-aiding audio signal updating method and apparatus, so that a sleep-aiding audio signal can be updated, to provide a proper sleep-aiding audio signal for a user, and ensure a sleep-aiding effect.

According to a first aspect, a sleep-aiding audio signal updating method is provided, including: obtaining a first biological signal collected when a first sleep-aiding audio signal in a sleep-aiding audio library is played, where the first biological signal is a biological signal of a first user; determining a sleep quality of the first user based on the first biological signal; and updating the sleep-aiding audio library based on the sleep quality of the first user.

Optionally, for example, the obtaining a first biological signal may include receiving the first biological signal; the obtaining a first biological signal may include collecting the first biological signal; or the obtaining a first biological signal may include obtaining the first biological signal entered by the user.

The sleep-aiding audio library includes a plurality of sleep-aiding audio signals. The first sleep-aiding audio signal is one of the plurality of sleep-aiding audio signals. The plurality of sleep-aiding audio signals may have a same time length or different time lengths. The plurality of sleep-aiding audio signals may be of a same type or different types. For example, one sleep-aiding audio signal may be one piece of white noise, or may be one piece of light music. A type and a time length of the sleep-aiding audio signal are not limited in this embodiment of this application.

Optionally, the first sleep-aiding audio signal may be a sleep-aiding audio signal randomly played in the sleep-aiding audio library. Alternatively, the first sleep-aiding audio signal may be a sleep-aiding audio signal ranked first in the sleep-aiding audio library. Alternatively, the first sleep-aiding audio signal may be a sleep-aiding audio signal selected by the user.

The first biological signal may be a bioelectrical signal of the first user, or may be a signal obtained after the bioelectrical signal is preprocessed. For example, the preprocessing may be filtering the bioelectrical signal.

For example, the bioelectrical signal may include an electroencephalogram signal, an electrooculogram signal, and an electromyogram signal.

The determining a sleep quality of the first user based on the first biological signal may include a plurality of manners. For example, total sleep duration of the first user is determined based on the first biological signal, and the sleep quality of the first user is determined based on the total sleep duration.

According to the solution in this embodiment of this application, the sleep-aiding audio signal may be updated based on the sleep quality of the user. That is, information related to sleep of the user is determined by using the biological signal; and then, the sleep quality of the user is evaluated, and a sleep-aiding effect of the sleep-aiding audio signal is evaluated based on the sleep quality of the user. Compared with updating an audio signal based on another parameter, the solution in this application can better meet a sleep quality requirement of the user and improve a sleep-aiding effect.

In addition, an evaluation that is of a sleep quality of a user and that is obtained by using a same sleep-aiding audio signal may be continuously updated, so that accuracy of evaluating a sleep-aiding effect of the sleep-aiding audio signal can be improved.

With reference to the first aspect, in some implementations of the first aspect, the determining a sleep quality of the first user based on the first biological signal includes: determining at least one of a plurality of sleep stages based on the first biological signal; and determining the sleep quality of the first user based on the at least one sleep stage.

The plurality of sleep stages are sleep stages obtained by performing sleep period division on a sleep process. Sleep stage division may be performed in different manners. For example, the plurality of sleep stages include a W period, a REM period, an N1 period, an N2 period, an N3 period, and an N4 period. For another example, the plurality of sleep stages include a W period, a REM period, an LS period, and an SWS period.

Optionally, the determining the sleep quality of the first user based on the at least one sleep stage may include: determining the sleep quality of the first user based on duration of the at least one sleep stage. For example, the at least one sleep stage includes a first sleep stage, and the sleep quality of the first user is determined based on duration of the first sleep stage. When the duration of the first sleep stage is longer, the sleep quality of the first user is better.

It should be understood that the duration of the sleep stage in this embodiment of this application may be actual duration, or may be a proportion of the duration of the sleep stage in total sleep duration.

According to the solution in this embodiment of this application, the sleep quality of the user is determined based on the sleep stage of the user, so that impact of different sleep stages on the sleep quality of the user can be fully considered, thereby improving accuracy of evaluating the sleep quality of the user. For example, deep sleep has relatively large impact on human mental and physical strength, and a whole sleep quality of the user can be more accurately evaluated by using a sleep stage related to the deep sleep.

With reference to the first aspect, in some implementations of the first aspect, the determining a sleep quality of the first user based on the first biological signal includes: determining at least one of a plurality of sleep stages based on the first biological signal; and determining, based on the at least one sleep stage, a sleep quality corresponding to the at least one sleep stage.

Optionally, the determining, based on the at least one sleep stage, a sleep quality corresponding to the at least one sleep stage includes: determining, based on duration of the at least one sleep stage, the sleep quality corresponding to the at least one sleep stage. For example, the at least one sleep stage includes a first sleep stage, and a sleep quality corresponding to the first sleep stage is determined based on duration of the first sleep stage. When the duration of the first sleep stage is longer, the sleep quality corresponding to the first sleep stage is better.

According to the solution in this embodiment of this application, sleep qualities of different sleep stages are determined based on the different sleep stages of the user, so that the sleep qualities of the different sleep stages can be fully considered, thereby improving accuracy of evaluating the sleep quality of the user.

With reference to the first aspect, in some implementations of the first aspect, the sleep-aiding audio library includes sleep-aiding audio signals corresponding to the plurality of sleep stages; and the updating the sleep-aiding audio library based on the sleep quality of the first user includes: updating, based on the sleep quality corresponding to the at least one sleep stage, a sleep-aiding audio signal corresponding to the at least one sleep stage in the sleep-aiding audio library, to obtain an updated sleep-aiding audio signal corresponding to the at least one sleep stage.

According to the solution in this embodiment of this application, sleep-aiding audio signals corresponding to the different sleep stages are updated based on the sleep qualities of the different sleep stages, so that diversity of the sleep-aiding audio signals in the sleep-aiding audio library can be improved, sleep-aiding requirements of the different sleep stages can be met, and a sleep-aiding effect of the sleep-aiding audio signal can be improved.

With reference to the first aspect, in some implementations of the first aspect, the method further includes: determining a target sleep-aiding audio signal based on the updated sleep-aiding audio signal corresponding to the at least one sleep stage, where the target sleep-aiding audio signal is used to be played for the first user when the first user is in the at least one sleep stage.

According to the solution in this embodiment of this application, corresponding target sleep-aiding audio signals may be played for the user in the different sleep stages, thereby meeting sleep-aiding requirements of the different sleep stages and improving a sleep-aiding effect of the sleep-aiding audio signal.

With reference to the first aspect, in some implementations of the first aspect, the determining, based on the at least one sleep stage, a sleep quality corresponding to the at least one sleep stage includes: determining, based on the duration of the at least one sleep stage and a reference value corresponding to the at least one sleep stage, the sleep quality corresponding to the at least one sleep stage.

For example, the sleep quality corresponding to the first sleep stage is determined based on a difference between the duration of the first sleep stage and a first reference value. The first reference value is a reference value corresponding to the first sleep stage.

Specifically, the sleep quality corresponding to the first sleep stage may be evaluated by using an absolute value of the difference between the duration of the first sleep stage and the first reference value. A lower absolute value of the difference indicates a better sleep quality corresponding to the first sleep stage.

For different users, a same sleep stage may correspond to a same reference value or different reference values.

For example, the reference value may be determined based on an age stage of a user. That is, for users of different age stages, a same sleep stage may correspond to a same reference value or different reference values.

For example, the reference value may be determined based on a gender of a user. That is, for users of different genders, a same sleep stage may correspond to different reference values.

With reference to the first aspect, in some implementations of the first aspect, the method further includes: obtaining feedback information of the first user for the first sleep-aiding audio signal; and updating, based on the feedback information, the reference value corresponding to the at least one sleep stage.

The feedback information of the user for the sleep-aiding audio signal is feedback information of the user for a current sleep quality, namely, an evaluation of the user for a sleep quality in a case in which the sleep-aiding audio signal is played.

In actual life, all persons differ in sleep status, and a sleep quality evaluation result obtained based on a fixed parameter value is not necessarily suitable for all the persons. A result of evaluating the current sleep quality based on a feeling of the user may be inconsistent with a result of determining the sleep quality based on a fixed parameter value. For example, it may be determined, based on the feeling of the user, that the current sleep quality is not good, the user feels dizzy and tired after waking up, and so on. However, when the current sleep quality is evaluated based on the fixed reference value, it may be determined that the current sleep quality is very good. In this case, a sleep quality obtained through determining based on the fixed reference value is no longer accurate. According to the solution in this embodiment of this application, the feedback information of the user for the current sleep quality may be obtained, that is, the evaluation of the user for the current sleep quality may be obtained; and then, a sleep quality evaluation manner is updated based on this, to improve accuracy of evaluating the sleep quality of the user, thereby improving a sleep-aiding effect of the sleep-aiding audio signal.

With reference to the first aspect, in some implementations of the first aspect, the updating the sleep-aiding audio library based on the sleep quality of the first user includes: updating a sequence of the sleep-aiding audio signals in the sleep-aiding audio library based on the sleep quality of the first user; and/or deleting one or more sleep-aiding audio signals from the sleep-aiding audio library based on the sleep quality of the first user.

For example, the sleep-aiding audio signals in the sleep-aiding audio library may be sorted by using a method such as bubble sort, selection sort, insertion sort, merge sort, or quick sort. A specific sorting form is not limited in this embodiment of this application.

Optionally, the deleting one or more sleep-aiding audio signals from the sleep-aiding audio library based on the sleep quality of the first user may include: deleting, based on a sleep quality corresponding to at least one sleep stage of the first user, one or more sleep-aiding audio signals from a plurality of sleep-aiding audio signals corresponding to the at least one sleep stage. For example, a sleep-aiding audio signal ranked low may be deleted.

With reference to the first aspect, in some implementations of the first aspect, the first sleep-aiding audio signal is a newly added sleep-aiding audio signal.

The newly added sleep-aiding audio signal is a sleep-aiding audio signal that has no corresponding sleep quality of the first user. For example, the newly added sleep-aiding audio signal may be a sleep-aiding audio signal uploaded to the sleep-aiding audio library for the first time. The newly added sleep-aiding audio signal may be a sleep-aiding audio signal added by a system, or may be a sleep-aiding audio signal added by the user.

According to the solution in this embodiment of this application, a sleep-aiding audio signal can be added to the sleep-aiding audio library, a sleep quality of the user in a case in which the newly-added sleep-aiding audio signal is played can be determined, and the sleep-aiding audio library can be updated based on the sleep quality, thereby helping add a sleep-aiding audio signal with a better sleep-aiding effect.

With reference to the first aspect, in some implementations of the first aspect, the method further includes: determining a sleep quality of a second user, where the sleep quality of the second user is determined based on a second biological signal, the second biological signal is a biological signal of the second user, and the second biological signal is collected when a second sleep-aiding audio signal in the sleep-aiding audio library is played; and the updating the sleep-aiding audio library based on the sleep quality of the first user includes: updating the sleep-aiding audio library based on the sleep quality of the first user and the sleep quality of the second user.

Optionally, a process of determining the sleep quality of the second user may be the same as the process of determining the sleep quality of the first user.

Optionally, the determining a sleep quality of a second user may include: receiving the sleep quality of the second user from another device.

According to the solution in this embodiment of this application, the sleep-aiding audio library can be updated based on sleep qualities of a plurality of users, thereby improving accuracy of updating the sleep-aiding audio signal, and helping improve a sleep-aiding effect.

According to a second aspect, a sleep-aiding audio signal updating apparatus is provided, including: an obtaining unit, configured to obtain a first biological signal collected when a first sleep-aiding audio signal in a sleep-aiding audio library is played, where the first biological signal is a biological signal of a first user; and a processing unit, configured to: determine a sleep quality of the first user based on the first biological signal; and update the sleep-aiding audio library based on the sleep quality of the first user.

According to the solution in this embodiment of this application, the sleep-aiding audio signal may be updated based on the sleep quality of the user. That is, information related to sleep of the user is determined by using the biological signal; and then, the sleep quality of the user is evaluated, and a sleep-aiding effect of the sleep-aiding audio signal is evaluated based on the sleep quality of the user. Compared with updating an audio signal based on another parameter, the solution in this application can better meet a sleep quality requirement of the user and improve a sleep-aiding effect.

In addition, an evaluation that is of a sleep quality of a user and that is obtained by using a same sleep-aiding audio signal may be continuously updated, so that accuracy of evaluating a sleep-aiding effect of the sleep-aiding audio signal can be improved.

With reference to the second aspect, in some implementations of the second aspect, the processing unit is specifically configured to: determine at least one of a plurality of sleep stages based on the first biological signal; and determine, based on the at least one sleep stage, a sleep quality corresponding to the at least one sleep stage.

With reference to the second aspect, in some implementations of the second aspect, the sleep-aiding audio library includes sleep-aiding audio signals corresponding to the plurality of sleep stages; and the processing unit is specifically configured to update, based on the sleep quality corresponding to the at least one sleep stage, a sleep-aiding audio signal corresponding to the at least one sleep stage in the sleep-aiding audio library, to obtain an updated sleep-aiding audio signal corresponding to the at least one sleep stage.

With reference to the second aspect, in some implementations of the second aspect, the processing unit is further configured to determine a target sleep-aiding audio signal based on the updated sleep-aiding audio signal corresponding to the at least one sleep stage, where the target sleep-aiding audio signal is used to be played for the first user when the first user is in the at least one sleep stage.

With reference to the second aspect, in some implementations of the second aspect, the processing unit is specifically configured to determine, based on duration of the at least one sleep stage and a reference value corresponding to the at least one sleep stage, the sleep quality corresponding to the at least one sleep stage.

With reference to the second aspect, in some implementations of the second aspect, the processing unit is further configured to: obtain feedback information of the first user for the first sleep-aiding audio signal; and update, based on the feedback information, the reference value corresponding to the at least one sleep stage.

With reference to the second aspect, in some implementations of the second aspect, the processing unit is specifically configured to: update a sequence of the sleep-aiding audio signals in the sleep-aiding audio library based on the sleep quality of the first user; and/or delete one or more sleep-aiding audio signals from the sleep-aiding audio library based on the sleep quality of the first user.

With reference to the second aspect, in some implementations of the second aspect, the first sleep-aiding audio signal is a newly added sleep-aiding audio signal.

With reference to the second aspect, in some implementations of the second aspect, the processing unit is further configured to determine a sleep quality of a second user, where the sleep quality of the second user is determined based on a second biological signal, the second biological signal is a biological signal of the second user, and the second biological signal is collected when a second sleep-aiding audio signal in the sleep-aiding audio library is played; and the processing unit is specifically configured to update the sleep-aiding audio library based on the sleep quality of the first user and the sleep quality of the second user.

According to a third aspect, a sleep-aiding audio signal updating apparatus is provided, including an input/output interface, a processor, and a memory. The processor is configured to control the input/output interface to receive/send information. The memory is configured to store a computer program. The processor is configured to invoke the computer program from the memory and run the computer program, to enable the apparatus to perform the method in the first aspect.

Optionally, the apparatus may be a terminal device/server, or may be a chip in the terminal device/server.

Optionally, the memory may be located inside the processor, for example, may be a cache (cache) in the processor. The memory may be alternatively located outside the processor, to be independent of the processor, for example, may be an internal memory (memory) of the apparatus.

According to a fourth aspect, a computer program product is provided. The computer program product includes computer program code. When the computer program code is run on a computer, the computer is enabled to perform the method in the first aspect.

It should be noted that all or a part of the computer program code may be stored in a first storage medium. The first storage medium may be encapsulated together with a processor, or may be encapsulated separately from a processor. This is not specifically limited in this embodiment of this application.

According to a fifth aspect, a computer readable medium is provided. The computer readable medium stores program code. When the computer program code is run on a computer, the computer is enabled to perform the method in the first aspect.

According to a sixth aspect, a chip is provided. The chip includes a processor and a data interface. The processor reads, by using the data interface, instructions stored in a memory, to perform the method in the first aspect.

Optionally, as an implementation, the chip may further include the memory. The memory stores the instructions. The processor is configured to execute the instructions stored in the memory. When the instructions are executed, the processor is configured to perform the methods in the first aspect.

It should be understood that the first aspect includes any implementation of the first aspect.

**BRIEF DESCRIPTION OF DRAWINGS**

FIG. 1 is a schematic block diagram of sleep period division;
FIG. 2 is a schematic diagram of brain waves in different sleep stages;
FIG. 3 is a schematic diagram of a cycle distribution status of sleep stages in a sleep process;
FIG. 4 is a schematic block diagram of a sleep-aiding audio signal updating system according to an embodiment of this application;
FIG. 5 is a schematic flowchart of a sleep-aiding audio signal updating method according to an embodiment of this application;
FIG. 6 is a schematic diagram of mapping relationships between sleep-aiding audio signals and sleep qualities according to an embodiment of this application;
FIG. 7A and FIG. 7B are a schematic diagram of an integration result of different sleep-aiding audio signals according to an embodiment of this application;
FIG. 8A and FIG. 8B are a schematic diagram of a sleep-aiding audio signal sorting result according to an embodiment of this application;
FIG. 9 is a schematic block diagram of a sleep-aiding audio signal updating system according to an embodiment of this application;
FIG. 10 is a schematic block diagram of another sleep-aiding audio signal updating system according to an embodiment of this application;
FIG. 11 is a schematic block diagram of a sleep-aiding audio signal updating apparatus according to an embodiment of this application; and
FIG. 12 is a schematic diagram of a hardware structure of a sleep-aiding audio signal updating apparatus according to an embodiment of this application.

**DESCRIPTION OF EMBODIMENTS**

The following describes technical solutions of this application with reference to the accompanying drawings.

A sleep-aiding audio signal updating method provided in embodiments of this application can be applied to a sleep-aiding scenario. A sleep quality of a user is determined by analyzing a bioelectrical signal of the user, and a sleep-aiding audio signal is updated based on the sleep quality of the user.

For ease of understanding the embodiments of this application, the following first describes related concepts of related terms in the embodiments of this application.

(1) Bioelectrical signal

Bioelectricity is a regular electrical phenomenon that is closely related to a life status and that is produced by an active cell or tissue (human body or animal tissue) regardless of whether the active cell or tissue is in a static state or an active state.

The bioelectrical signal may be obtained from a skin surface of a human body. Relatively common bioelectrical signals include an electroencephalogram (EEG) signal, an electrocardiogram ECG signal, an electrooculogram EOG signal, an electromyogram EMG signal, and the like. Sleep period division can be implemented by analyzing the bioelectrical signal, to obtain different sleep stages. Sleep period division is usually implemented by analyzing an electroencephalogram signal. The following describes the electroencephalogram signal.

The electroencephalogram signal is an external reflection of a brain activity, and different brain activities are represented as electroencephalogram signals with different features. Studies show that performing spatial-temporal frequency domain analysis on these electroencephalogram signals is helpful for reversely analyzing a human intentional activity.

In this embodiment of this application, an electroencephalogram may also be referred to as a brain wave.

A scalp electroencephalogram signal is a bioelectrical signal. After an electric field formed due to the change of 86 billion neurons in a brain is conducted by a volumetric conductor including a cortex, a skull, a meninx, and a scalp, a potential distribution is formed on the scalp. An electroencephalogram signal can be obtained by recording these changed potential distributions.

Electroencephalogram signals may be classified into a spontaneous electroencephalogram and an evoked potential (EP). The spontaneous electroencephalogram is a potential change spontaneously generated by nerve cells in a brain without a specific external stimulus. The evoked potential is a potential change caused by nerve cells in a brain due to different types of stimuli such as sound, light, and electricity. Spontaneous potentials of different frequencies can reflect different human states. Table 1 shows a manner of classifying spontaneous electroencephalograms based on frequency ranges. As shown in Table 1, spontaneous electroencephalograms of different types can reflect different human states.

**Table 1**

| Brain wave | Brain wave type | | Frequency/Hz | Human state |
|---|---|---|---|---|
| Spontaneous potential | Delta ($\delta$) | | 0.1 to 3 | Deep sleep without a dream |
| | Theta ($\theta$) | | 4 to 7 | Adult emotional stress, such as disappointment or frustration |
| | Alpha ($\alpha$) | | 8 to 12 | Relaxed, calm, and closed but wakeful |
| | Beta ($\beta$) | L | 12.5 to 16 | Relaxed but focused |
| | | M | 16.5 to 20 | Thinking and processing a received external message |
| | | H | 20.5 to 28 | Agitated and anxious |
| | Gamma ($\gamma$) | | 25 to 100 | Awareness-raising, happiness-raising, stress relief, and meditation |

In an implementation, a brain wave obtaining manner may include: separately obtaining brain waves at positions such as an occipital, a parietal occipital, a parietal lobe, a pituitary gland, and a forehead. For example, a headgear is worn on a head of a user, and a plurality of brain wave collection modules are disposed in the headgear. The plurality of brain wave collection modules correspond to the positions such as the occipital, the parietal occipital, the parietal lobe, the pituitary gland, and the forehead, to measure the brains wave at the positions.

(2) Sleep period division

From wakefulness to sleep is not a momentary transition between two states, but a brief transition stage. In a sleep process, a sleep state is not stable or unchanged, but a plurality of different sleep stages alternate over and over again. Sleep period division based on an electroencephalogram signal indicates that several different sleep stages in a sleep process are separated to determine a time length occupied by each sleep stage, and then a sleep quality can be analyzed.

*A Manual of Standardized Terminology, Techniques and Scoring System for Sleep Stages of Human Subjects,* referred to as the R&K standard, formulated by Rechtschaffen and Kales in 1968, becomes a standard for sleep period division work. In this standard, a sleep process includes wakefulness (W), non-rapid eye movement sleep (non-rapid eye movement sleep, NREM), and rapid eye movement sleep (REM). The non-rapid eye movement sleep is further divided into four periods: an NREM sleep period 1 (N1), an NREM sleep period 2 (N2), an NREM sleep period 3 (N3), and an NREM sleep period 4 (N4).

In one time of complete sleep, different sleep stages have different amplitude and frequency features. The following describes in detail features of EEG signals in the foregoing six different sleep stages.

Wakefulness period (W): In most cases, a person is in a wakefulness period and in a state of constantly sensing and responding to external stimuli. Visual stimuli, auditory stimuli, thinking activities, and mental activities are most active, and brain activities are most complex. Compared with an electroencephalogram signal in a sleep period, an electroencephalogram signal in the wakefulness period is characterized by a low amplitude and a high frequency, where an amplitude is usually less than 50 μV, and a frequency range is 1.5 Hz to 50 Hz. A strong electrooculogram signal can be observed from a collected electroencephalogram signal. This has great impact on electroencephalogram signal processing. Therefore, in terms of electroencephalogram signal processing, artifact removal usually needs to be performed on the electroencephalogram signal collected in the wakefulness period, to reduce electrooculogram interference.

NREM sleep period 1 (N1): In the N1 period, physical activities of the person start to decrease, a mind of the person starts to be confused, and consciousness of the person gradually blurs. After a pre-sleep period of a few minutes, a brain state gradually stabilizes, and time of the whole period is about 1 min to 10 min. In this period, the person is prone to be awakened, and the awakened person usually denies sleep. In terms of physiological indexes, an electromyogram level significantly decreases, a heart rate significantly becomes slow, a blood pressure and a body temperature slightly decrease compared with those in a wakefulness state, and respiration is gradually regular. An electroencephalogram signal is a low-voltage mixed wave, a main frequency is 4 Hz to 8 Hz, and an amplitude is 50 μV to 100 μV. A spike signal may appear, but no spindle or K-complex appears.

NREM sleep period 2 (N2): It is generally considered that real sleep starts from the N2 period, and the period lasts for 10 min to 20 min. Both the N1 period and the N2 period are light sleep periods, where the person may be awakened, or may wake. In the N2 stage, a spindle and a K-complex appear in the brain, a main frequency is 4 Hz to 15 Hz, and an amplitude is 50 μV to 150 μV. The main frequency and the amplitude are slightly greater than those in the sleep period 1. A cycle in which the spindle or the K-complex appears is generally less than 3 minutes, otherwise it is considered that the sleep period 2 has not been entered yet.

NREM sleep period 3 (N3): The appearance of this period indicates that the person starts to enter deep sleep, the consciousness disappears, and it is difficult to awaken the person. An electroencephalogram signal is mainly a slow wave, a frequency is 2 Hz to 4 Hz, and an amplitude is 100 μV to 150 μV. A spindle and a K-complex may also appear in this period.

NREM sleep period 4 (N4): N4 sleep is relatively deep, and wakening is very difficult. Features of an electroencephalogram signal are similar to those in the sleep period 3, but components below 2 Hz obviously increase, a frequency is mainly 0.5 Hz to 2 Hz, and an amplitude is between 100 μV to 200 μV.

REM sleep period: In this period, it can be found that eyeballs rapidly move, but the body does not move. Most dreams occur in this period. REM sleep generally lasts for 90 minutes to 120 minutes, and the NREM sleep generally lasts for 4 hours to 7 hours. The REM sleep lasts for relatively short time, but plays an important role in a human memory function. An electroencephalogram signal is mainly a mixed low-pressure fast wave, a frequency is 15 Hz to 30 Hz, and an amplitude is usually less than 50 μV.

Currently, a segment of data may be extracted from each sleep stage of a testee based on a manual period division result of an expert, to perform a feature analysis. A test object A is used as an example. Sleep time of the test object A is 380 minutes, and a length of each set of data is 7500 points (a sampling frequency is 250 Hz, and collection lasts for 30s). Data of 25 minutes is selected from each sleep stage to perform feature extraction, and energy ratios of an alpha wave, a beta wave, a theta wave, a delta wave, and an electromyogram (EMG) high-frequency component of each sleep stage are calculated. Specifically, wavelet decomposition is performed by using a Daubechies db4 wavelet base; D3 is selected to represent a beta wave, D4 is selected to represent an alpha wave, D5 is selected to represent a theta wave, and D6+D7 is selected to represent a delta wave; and ratios of energy of the alpha wave (8 Hz to 13 Hz), the beta wave (13 Hz to 30 Hz), the theta wave (4 Hz to 7 Hz), and the delta wave (1 Hz to 4 Hz) in an energy sum in 1 Hz to 30 Hz are separately calculated. The energy ratios meet the following formula (1).

$$\mu_i = \frac{\sum_{k=1}^{n}|D_i(k)|^2}{E_S} \tag{1}$$

, where

$$E_S = \sum_{k=1}^{N}|D_i(k)|^2$$

$\mu_i$ is a ratio that is of energy of a frequency band of an $i^{th}$ layer in the energy sum and that is obtained after the decomposition, $D_i(k)$ is a $k^{th}$ wavelet coefficient that is at the $i^{th}$ layer and that is obtained after the decomposition, n is a quantity of pieces of data at the $i^{th}$ layer, $E_S$ is the energy sum of frequency bands of layers, and N is a quantity of pieces of data of all the layers.

According to the foregoing method, feature parameters of each sleep stage, namely, the energy ratios of the alpha wave, the beta wave, the theta wave, the delta wave, and the EMG high-frequency component of each sleep stage, may be obtained through calculation; and then, an average value of each of these feature parameters in all sleep stages may be obtained through calculation, to obtain numerical features of each feature parameter in different sleep stages. For example, the feature parameters are shown in Table 2. Therefore, sleep period division can be implemented by analyzing the feature parameters.

**Table 2**

| Feature parameter | W | N1 | N2 | N3 | REM |
|---|---|---|---|---|---|
| Alpha wave | 0.1837 | 0.1050 | 0.0702 | 0.0240 | 0.0937 |
| Beta wave | 0.1170 | 0.0768 | 0.0286 | 0.0085 | 0.0508 |
| Theta wave | 0.0802 | 0.0806 | 0.0671 | 0.0350 | 0.1210 |
| Delta wave | 0.1214 | 0.1860 | 0.3312 | 0.3910 | 0.2687 |
| EMG high-frequency component | 0.1163 | 0.0863 | 0.0331 | 0.0805 | 0.0133 |
| EEG sample entropy | 1.3060 | 1.0451 | 0.5054 | 0.3827 | 0.7550 |

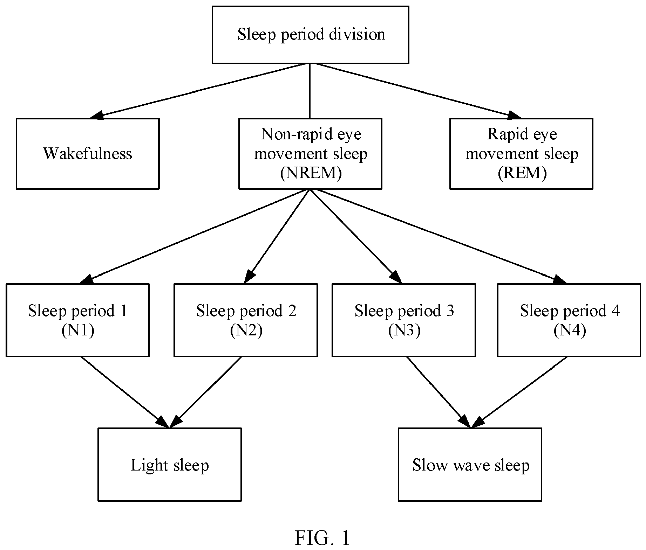

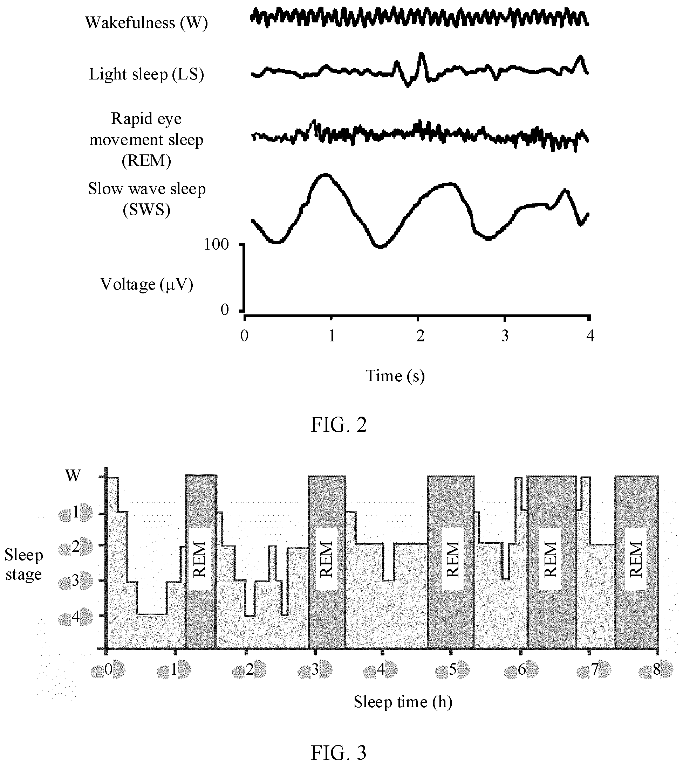

In 2007, the American Academy of Sleep Medicine integrated the R&K standard and proposed a revised version. The revised version is supported by most sleep centers in Europe and the United States. This standard has also been adopted in China. In the revised R&K sleep period division standard, N1 and N2 are combined into light sleep (LS), and N3 and N4 are combined into slow wave sleep (SWS). FIG. 1 is a schematic diagram of a new sleep period division structure. FIG. 2 is a schematic diagram of brain waves in different sleep stages. FIG. 3 shows a cycle distribution status of sleep stages in a sleep process of a common person.

(3) Convolutional neural network

A convolutional neural network (CNN) is a deep neural network with a convolutional structure. The convolutional neural network includes a feature extractor including a convolutional layer and a sampling sublayer, and the feature extractor may be considered as a filter. The convolutional layer is a neuron layer that is in the convolutional neural network and at which convolution processing is performed on an input signal. In the convolutional layer of the convolutional neural network, one neuron may be connected to only some of neighboring layer neurons. One convolutional layer usually includes several feature planes, and each feature plane may include some rectangularly-arranged neurons. Neurons in a same feature plane share a weight, and the shared weight herein is a convolution kernel. The shared weight may be understood as that a manner of extracting image information is independent of a position. The convolution kernel may be initialized in a form of a matrix of a random size. In a training process of the convolutional neural network, a proper weight may be obtained through learning for the convolution kernel. In addition, a direct benefit of the shared weight is reducing connections between layers of the convolutional neural network, and also reducing an overfitting risk.

(4) Loss function

In a process of training a deep neural network, because it is expected that an output of the deep neural network is as much as possible close to a predicted value that is actually expected, a predicted value of a current network and a target value that is actually expected may be compared, and then a weight vector of each layer of the neural network is updated based on a difference between the predicted value and the target value (certainly, there is usually an initialization process before a first update, to be specific, parameters are preconfigured for all layers of the deep neural network). For example, if the predicted value of the network is large, the weight vector is adjusted to decrease the predicted value, and adjustment is continuously performed, until the deep neural network can predict the target value that is actually expected or a value that is very close to the target value that is actually expected. Therefore, "how to obtain, through comparison, a difference between the predicted value and the target value" needs to be predefined. This is a loss function (loss function) or an objective function (objective function). The loss function and the objective function are important equations that measure the difference between the predicted value and the target value. The loss function is used as an example. A higher output value (loss) of the loss function indicates a larger difference. Therefore, training of the deep neural network is a process of minimizing the loss as much as possible.

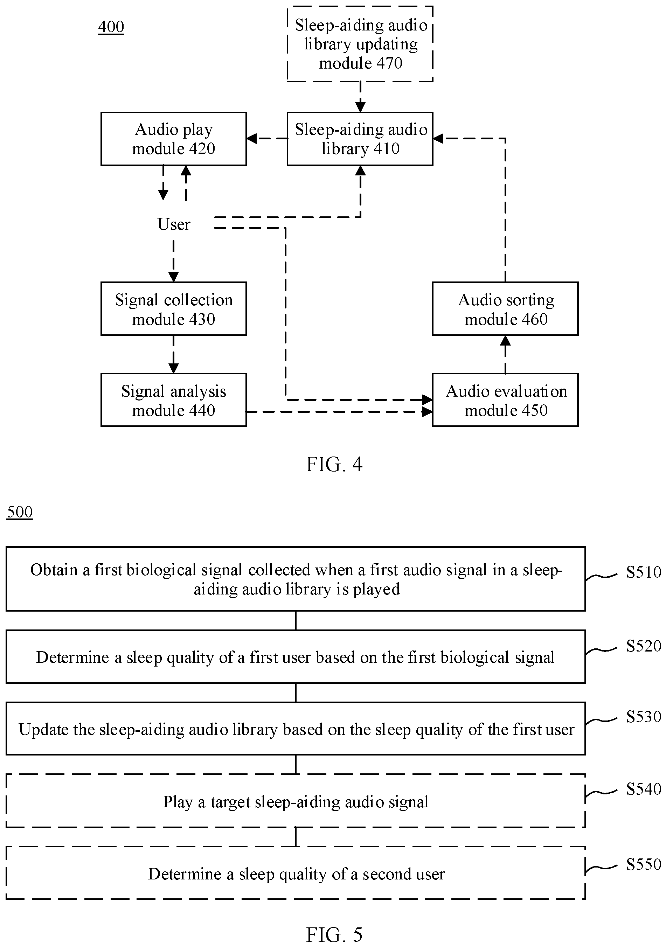

FIG. 4 is a schematic block diagram of a sleep-aiding audio signal updating system according to an embodiment of this application. A sleep-aiding audio signal updating system 400 in FIG. 4 includes a sleep-aiding audio library 410, an audio play module 420, a signal collection module 430, a signal analysis module 440, an audio evaluation module 450, an audio sorting module 460, and a sleep-aiding audio library updating module 470.

The sleep-aiding audio library 410 is configured to store a sleep-aiding audio signal. The sleep-aiding audio library 410 may include a plurality of sleep-aiding audio signals.

For example, a sequence of the plurality of sleep-aiding audio signals may be determined by the audio sorting module 460.

For example, a sequence of the plurality of sleep-aiding audio signals may be determined based on a user preference and the audio sorting module 460. For example, the audio sorting module 460 may sort the plurality of sleep-aiding audio signals to obtain a first sorting result, and send the first sorting result to the sleep-aiding audio library 410. The sleep-aiding audio library 410 may adjust the first sorting result based on the user preference, obtain a second sorting result, and use the second sorting result as the sequence of the plurality of sleep-aiding audio signals.

The audio play module 420 is configured to play a sleep-aiding audio signal in the sleep-aiding audio library 410.

For example, the audio play module 420 may play a sleep-aiding audio signal in the sleep-aiding audio library based on the sequence of the plurality of sleep-aiding audio signals in the sleep-aiding audio library 410.

For example, the audio play module 420 may play a sleep-aiding audio signal based on the user preference. For example, the audio play module 420 may play a sleep-aiding audio signal selected by a user.

The signal collection module 430 is configured to: collect a bioelectrical signal, for example, a brain wave signal, of the user, and transmit the bioelectrical signal to the signal analysis module 440.

Optionally, the signal collection module may preprocess the bioelectrical signal, for example, filter the bioelectrical signal, to obtain a biological signal; and transmit the biological signal to the signal analysis module 440.

The signal analysis module 440 is configured to process or analyze data transmitted by the signal collection module, to determine a sleep quality of the user.

The audio evaluation module 450 is configured to evaluate/score the sleep-aiding audio signal based on the sleep quality of the user.

For example, a mapping relationship is established between the sleep-aiding audio signal and a sleep quality corresponding to the sleep-aiding audio signal, and the sleep quality of the user is used as a score of the sleep-aiding audio signal. The sleep quality corresponding to the sleep-aiding audio signal is a sleep quality that is of the user and that is determined by using a bioelectrical signal collected when the sleep-aiding audio signal is played.

Further, the audio evaluation module 450 may score the sleep-aiding audio signal based on the sleep quality of the user.

Optionally, the audio evaluation module 450 may score the sleep-aiding audio signal based on the user preference, for example, score the sleep-aiding audio signal based on a quantity of selection times of the user and the sleep quality of the user.

The audio sorting module 460 is configured to sort the sleep-aiding audio signals in the sleep-aiding audio library based on the score of the sleep-aiding audio signal. Alternatively, the audio sorting module 460 may determine a sleep-aiding audio signal with a highest score from the sleep-aiding audio library 410.

The sleep-aiding audio library updating module 470 is configured to add a sleep-aiding audio signal to the sleep-aiding audio library or delete a sleep-aiding audio signal from the sleep-aiding audio library. For example, the user may add one or several sleep-aiding audio signals to the sleep-aiding audio library or delete one or several sleep-aiding audio signals from the sleep-aiding audio library by using the sleep-aiding audio library updating module 470. For another example, the system may add a sleep-aiding audio signal to the sleep-aiding audio library 410.

With reference to FIG. 5, the following describes in detail a sleep-aiding audio signal updating method provided in the embodiments of this application.

FIG. 5 shows a sleep-aiding audio signal updating method 500 according to an embodiment of this application. For example, the sleep-aiding audio signal updating method 500 may be performed by the sleep-aiding audio signal updating system shown in FIG. 4. The method 500 includes step S510 to step S550. The following describes step S510 to step S550 in detail.

S510: Obtain a first biological signal collected when a first audio signal in a sleep-aiding audio library is played.

Specifically, the first sleep-aiding audio signal in the sleep-aiding audio library is played for a first user, the first biological signal is collected when the first sleep-aiding audio signal is played, and the collected first biological signal is obtained. The first biological signal is a biological signal of the first user.

For example, the obtaining a first biological signal may include receiving the first biological signal; the obtaining a first biological signal may include collecting the first biological signal; or the obtaining a first biological signal may include obtaining the first biological signal entered by the user.

The sleep-aiding audio library includes a plurality of sleep-aiding audio signals. The first sleep-aiding audio signal is one of the plurality of sleep-aiding audio signals. The plurality of sleep-aiding audio signals may have a same time length or different time lengths. The plurality of sleep-aiding audio signals may be of a same type or different types. For example, one sleep-aiding audio signal may be one piece of white noise, or may be one piece of light music. A type and a time length of the sleep-aiding audio signal are not limited in this embodiment of this application.

For example, the sleep-aiding audio library may be the sleep-aiding audio library 410 in FIG. 4. The playing the first sleep-aiding audio signal in the sleep-aiding audio library for a first user may be performed by the audio play module 420 in FIG. 4.

For example, the audio play module 420 may be located in a device such as a wearable device, a mobile terminal, or a sound box. For example, the wearable device may include a headset. For another example, the mobile terminal may include a mobile phone or a tablet computer.

For example, if the sleep-aiding audio signal in the sleep-aiding audio library is played for the first user for the first time, a sequence of the plurality of sleep-aiding audio signals in the sleep-aiding audio library may be a random sequence. Alternatively, the sequence of the plurality of sleep-aiding audio signals in the sleep-aiding audio library may be determined by the audio sorting module 460 in FIG. 4.

For example, the first sleep-aiding audio signal may be a sleep-aiding audio signal randomly played in the sleep-aiding audio library. Alternatively, the first sleep-aiding audio signal may be determined based on the sequence of the plurality of sleep-aiding audio signals, for example, may be a sleep-aiding audio signal ranked first in the sleep-aiding audio library. Alternatively, the first sleep-aiding audio signal may be a sleep-aiding audio signal selected by the user.

For example, the collecting the first biological signal when the first sleep-aiding audio signal is played may be performed by the signal collection module 430 in FIG. 4.

The first biological signal may be a bioelectrical signal of the first user, or may be a bioelectrical signal obtained after the signal collection module 430 performs preprocessing. For example, the preprocessing may be filtering the bioelectrical signal.

For example, the bioelectrical signal may include an electroencephalogram signal, an electrooculogram signal, and an electromyogram signal.

For example, the signal collection module 430 may be located in a head-mounted device. For example, the head-mounted device may include a head cover, an eye cover, a headset, or a pillow.

S520: Determine a sleep quality of the first user based on the first biological signal.

For example, step S520 may be performed by the signal analysis module 440 in FIG. 4.

Step S520 may include: determining at least one of a plurality of sleep stages based on the first biological signal; and determining the sleep quality of the first user based on the at least one sleep stage.

The plurality of sleep stages are sleep stages obtained by performing sleep period division on a sleep process. Sleep stage division may be performed in different manners. For example, the plurality of sleep stages include a W period, a REM period, an N1 period, an N2 period, an N3 period, and an N4 period. For another example, the plurality of sleep stages include a W period, a REM period, an LS period, and an SWS period.

For example, specific processing, for example, decomposition and feature extraction, may be performed on the first biological signal, to obtain first processed signals, and sleep stages are determined based on the first processed signals.

For example, an independent components analysis (ICA) method is used to decompose the first biological signal to obtain source signal components of the first biological signal. These components may respectively correspond to an electroencephalogram signal, an electrooculogram signal, an electromyogram signal, and the like. Therefore, the electroencephalogram signal, the electrooculogram signal, the electromyogram signal, and the like are obtained through decomposition.

For another example, an energy sum ratio analysis method in a frequency domain analysis method may be further used to perform feature extraction on decomposed signals (for example, the electroencephalogram signal, the electrooculogram signal, and the electromyogram signal), to obtain feature signals.

That is, the first processed signals may be the decomposed signals obtained after the first biological signal is decomposed, may be the feature signals obtained after feature extraction is performed on the decomposed signals, or may be the first biological signal.

For example, sleep period division may be performed by using the foregoing sleep period division method, to determine sleep stages. It should be understood that the sleep period division method herein is merely an example, a specific form of the sleep period division method is not limited in this embodiment of this application, and another method that can determine sleep stages is also applicable to the method in this embodiment of this application.

For example, a sleep quality of a user may include a whole sleep quality of the user and/or a fragmented sleep quality of the user.

The whole sleep quality is a sleep quality in a whole sleep process. The fragmented sleep quality is a sleep quality corresponding to at least one of a plurality of sleep stages in the whole sleep process.

The following describes a method for determining a fragmented sleep quality of the first user.

Optionally, a sleep quality corresponding to at least one sleep stage may be determined based on the at least one sleep stage.

For example, the sleep quality corresponding to the at least one sleep stage may be determined based on duration of the at least one sleep stage. For example, the at least one sleep stage includes a first sleep stage, and a sleep quality corresponding to the first sleep stage is determined based on duration of the first sleep stage. When the duration of the first sleep stage is longer, the sleep quality corresponding to the first sleep stage is better.

It should be understood that the duration of the sleep stage in this embodiment of this application may be actual duration, or may be a proportion of the duration of the sleep stage in total sleep duration.

For example, the sleep quality corresponding to the at least one sleep stage may be determined based on the duration of the at least one sleep stage and a reference value corresponding to the at least one sleep stage.

For example, the sleep quality corresponding to the first sleep stage is determined based on a difference between the duration of the first sleep stage and a first reference value. The first reference value is a reference value corresponding to the first sleep stage.

Specifically, the sleep quality corresponding to the first sleep stage may be evaluated by using an absolute value of the difference between the duration of the first sleep stage and the first reference value. A lower absolute value of the difference indicates a better sleep quality corresponding to the first sleep stage.

The method for determining the fragmented sleep quality of the first user is described by using an example in which the sleep stages are classified into a W period, a REM period, an N1 period, an N2 period, an N3 period, and an N4 period.

For example, a sleep quality score of the W period meets the following:

$|W_i - W|$, where

$W_i$ represents duration of the W period, and $W$ represents a reference value corresponding to the W period. For example, the duration of the W period may be a proportion of actual duration of the W period in total sleep duration.

For example, a sleep quality score of the N1 period meets the following:

$|N1_i - N1|$, where

$N1_i$ represents duration of the N1 period, and $N1$ represents a reference value corresponding to the N1 period. For example, the duration of the N1 period may be a proportion of actual duration of the N1 period in the total sleep duration.

For example, a sleep quality score of the N2 period meets the following:

$|N2|_i - N2|$, where

$N2_i$ represents duration of the N2 period, and $N2$ represents a reference value corresponding to the N2 period. For example, the duration of the N2 period may be a proportion of actual duration of the N2 period in the total sleep duration.

For example, a sleep quality score of the N3 period meets the following:

$|N3_i - N3|$, where

$N3_i$ represents duration of the N3 period, and $N3$ represents a reference value corresponding to the N3 period. For example, the duration of the N3 period may be a proportion of actual duration of the N3 period in the total sleep duration.

For example, a sleep quality score of the N4 period meets the following:

$|N4_i - N4|$, where

$N4_i$ represents duration of the N4 period, and $N4$ represents a reference value corresponding to the N4 period. For example, the duration of the N4 period may be a proportion of actual duration of the N4 period in the total sleep duration.

For example, a sleep quality score of the REM period meets the following:

$|REM_i - REM|$, where

$REM_i$ represents duration of the REM period, and $REM$ represents a reference value corresponding to the REM period. For example, the duration of the REM period may be a proportion of actual duration of the REM period in the total sleep duration.

A lower sleep quality score indicates a better sleep quality.

For different users, a same sleep stage may correspond to a same reference value or different reference values.

For example, the reference value may be determined based on an age stage of a user. That is, for users of different age stages, a same sleep stage may correspond to different reference values.

Table 3 shows mapping relationships between different age stages and reference values. The reference value in Table 3 is represented in a form of a proportion of actual duration of a sleep stage in total sleep duration.

For example, the first sleep stage is the W period, and the first reference value is the reference value *W* corresponding to W. A reference value *W* corresponding to users of an age stage of 20 to 29 is 0.9, and a reference value *W* corresponding to users of an age stage of 40 to 59 is 4.1

**Table 3**

| Age stage<br>Reference value<br>Sleep stage | 20 to 29 | 30 to 39 | 40 to 59 | 60 or more |
|---|---|---|---|---|
| W | 0.9 | 2.4 | 4.1 | 9.9 |
| N1 | 5.3 | 7.5 | 10.9 | 11.9 |
| N2 | 48.7 | 53.0 | 51.1 | 50.6 |
| N3 | 7.7 | 5.5 | 3.4 | 4.5 |
| N4 | 13.2 | 9.6 | 7.7 | 2.7 |
| REM | 24.1 | 21.9 | 22.8 | 20.4 |

For example, the reference value may be determined based on a gender of a user. That is, for users of different genders, a same sleep stage may correspond to different reference values.

For example, the sleep quality corresponding to the at least one sleep stage may be determined by using a neural network model.

Specifically, sleep qualities may be classified. For example, sleep quality types include a high sleep quality, a common sleep quality, and a poor sleep quality.

A sleep quality classification model may be obtained through training in a machine learning manner. For example, the sleep quality classification model may be obtained by using a convolutional neural network (CNN).

Specifically, the sleep quality classification model is trained by using the convolutional neural network, that is, the first biological signal may be processed by using the convolutional neural network, to obtain a type of the sleep quality corresponding to the at least one sleep stage.

A sample biological signal and a label corresponding to the sample biological signal (for example, a sleep quality that is of at least one sleep stage and that has been determined by a doctor) are used as input of the convolutional neural network, and the label corresponding to the sample biological signal is used as target output of the convolutional neural network, to train the convolutional neural network.

When the sleep quality of the at least one sleep stage is determined by using the foregoing sleep quality classification model obtained through training, the first biological signal may be entered to the trained convolutional neural network to obtain the sleep quality of the at least one sleep stage.

The following describes a method for determining a whole sleep quality of the first user.

Example 1: The whole sleep quality of the first user is determined based on the total sleep duration.

Usually, human sleep duration falls within a specific duration range, for example, between 6 hours and 8 hours. When sleep duration is excessively short or excessively long, a rest effect of the user may be affected. Therefore, the whole sleep quality of the first user may be determined based on the total sleep duration.

Example 2: The whole sleep quality of the first user may be determined based on at least one sleep stage.

Specifically, the whole sleep quality of the user may be determined based on duration of the at least one sleep stage.

For example, the at least one sleep stage includes an N3 period and an N4 period, and the whole sleep quality of the first user is determined based on duration of the N3 period and duration of the N4 period.

Because deep sleep has relatively large impact on human mental and physical strength, when duration of N3 and N4 is relatively long, the whole sleep quality of the user is relatively good. The whole sleep quality of the user can be more accurately evaluated by using the duration of the N3 period and the N4 period.

Further, the whole sleep quality of the first user may be determined based on duration of at least two sleep stages and weights corresponding to the at least two sleep stages.

For example, the at least two sleep stage includes the REM period, the N1 period, the N2 period, the N3 period, and the N4 period. A whole sleep quality score S of the user may be calculated by using the following formula:

$$S = (REM + N3 + N4) \times a + N2 \times b + N1 \times c,$$

where

a, b, and c are weights of different sleep stages, and the weights may be set based on a requirement. For example, a=0.5, b=0.4, and c=0.1. A higher whole sleep quality score S indicates a better whole sleep quality.

For example, the whole sleep quality of the first user is determined based on a sleep quality corresponding to the at least one sleep stage.

That is, the whole sleep quality of the first user may be determined based on the fragmented sleep quality of the first user.

For example, the sleep quality corresponding to the at least one sleep stage may be determined based on the duration of the at least one sleep stage and a reference value corresponding to the at least one sleep stage, and then the whole sleep quality of the first user is determined based on the sleep quality corresponding to the at least one sleep stage. The method may be referred to as a linear sleep evaluation method, and a whole sleep quality obtained by using the method may be referred to as a whole linear sleep quality.

For example, the at least one sleep stage includes the W period, the REM period, the N1 period, the N2 period, the N3 period, and the N4 period. A whole sleep quality score S of the first user meets the following:

$$S = |W_i - W| + |N1_i - N1| + |N2_i - N2| + |N3_i - N3| + |N4_i - N4| + |REM_i - REM|$$

A smaller score value indicates a better whole sleep quality of the first user. In this way, impact of the fragmented sleep quality can be considered. The whole sleep quality of the user is evaluated based on the fragmented sleep quality, so that accuracy of evaluating the whole sleep quality can be improved.

For example, the whole sleep quality of the user is determined based on sleep qualities of at least two sleep stages and weights corresponding to the at least two sleep stages.

For example, the sleep qualities corresponding to the at least two sleep stages may be determined based on duration of the at least two sleep stages and reference values corresponding to the at least two sleep stages, and then the whole sleep quality of the first user is determined based on the sleep qualities corresponding to the at least two sleep stages. The method may be referred to as a weighted sleep evaluation method, and a whole sleep quality obtained by using the method may be referred to as a whole weighted sleep quality.

The at least two sleep stage includes the W period, the REM period, the N1 period, the N2 period, the N3 period, and the N4 period. A whole sleep quality score S of the first user meets the following:

$$S = k_W \times |W_i - W| + k_{N1} \times |N1_i - N1| + k_{N2} \times |N2_i - N2| + k_{N3} \times |N3_i - N3| +$$
$$k_{N4} \times |N4_i - N4| + k_{REM} \times |REM_i - REM|$$

A lower score indicates a better whole sleep quality of the user. $k_W$, $k_{N1}$, $k_{N2}$, $k_{N3}$, $k_{N4}$, and $k_{REM}$ respectively represent weights corresponding to the W period, the N1 period, the N2 period, the N3 period, the N4 period, and the REM period. $k_W$, $k_{N1}$, $k_{N2}$, $k_{N3}$, $k_{N4}$, and $k_{REM}$ meet $k_W + k_{N1} + k_{N2} + k_{N3} + k_{N4} + k_{REM} = 1$.

The whole sleep quality of the user is evaluated based on the weighted manner, so that weights of different sleep stages can be adjusted. Because deep sleep has relatively large impact on human mental and physical strength, a weight of a sleep stage corresponding to the deep sleep is increased, so that the impact of the deep sleep on the human mental and physical strength can be considered, thereby further improving accuracy of evaluating the whole sleep quality.

It should be understood that, in this embodiment of this application, the sleep quality evaluation method is described only in a sleep period division manner of the W period, the N1 period, the N2 period, the N3 period, the N4 period, and the REM period. A sleep period division manner is not limited in this embodiment of this application. A method for evaluating the sleep quality of the user based on another sleep period division manner is also applicable to the solution in this embodiment of this application.

Example 3: The whole sleep quality of the user may be determined by using a neural network model.

For example, a sleep quality classification model may be obtained through training in a machine learning manner, and the whole sleep quality of the user is determined by using the sleep quality classification model. For example, the sleep quality classification model may be obtained by using a convolutional neural network (CNN).

Specifically, the sleep quality classification model is trained by using the convolutional neural network, that is, the first biological signal may be processed by using the convolutional neural network, to obtain a type of the whole sleep quality of the first user.

A sample biological signal and a label corresponding to the sample biological signal (for example, a whole sleep quality that is of a user and that has been determined by a doctor) are used as input of the convolutional neural network, and the label corresponding to the sample biological signal is used as target output of the convolutional neural network, to train the convolutional neural network.

When the whole sleep quality of the user is determined by using the foregoing sleep quality classification model obtained through training, the first biological signal may be entered to the trained convolutional neural network to obtain the whole sleep quality of the first user.

In actual life, all persons differ in sleep status, and a sleep quality evaluation result obtained based on a fixed parameter value is not necessarily suitable for all the persons. A result of evaluating a current sleep quality based on a feeling of the user may be inconsistent with a result of determining the sleep quality based on a fixed reference value. For example, it may be determined, based on the feeling of the user, that the current sleep quality is not good, the user feels dizzy and tired after waking up, and so on. However, when the current sleep quality is evaluated based on the fixed reference value, it may be determined that the current sleep quality is very good. In this case, a sleep quality obtained through determining based on the fixed reference value is no longer accurate. According to the solution in this embodiment of this application, feedback information of the user for the current sleep quality may be obtained, that is, an evaluation of the user for the current sleep quality may be obtained; and then, a sleep quality evaluation manner is updated based on this, to improve accuracy of evaluating the sleep quality of the user, thereby improving a sleep-aiding effect of the sleep-aiding audio signal.

Optionally, the method 500 further includes: obtaining feedback information of the first user for the first sleep-aiding audio signal; and updating, based on the feedback information, a reference value corresponding to the at least one sleep stage.

The feedback information of the user for the sleep-aiding audio signal is feedback information of the user for a current sleep quality, namely, an evaluation of the user for a sleep quality in a case in which the sleep-aiding audio signal is played.

For example, when the user has a relatively high evaluation for the current sleep quality, a proportion of duration of each sleep stage in a current sleep cycle in total sleep duration is used as a reference value corresponding to each sleep period.

Optionally, the method 500 further includes: obtaining feedback information of the first user for the first sleep-aiding audio signal; and updating, based on the feedback information, a weight corresponding to the at least one sleep stage.

Further, step S520 may further include: determining the sleep quality of the first user based on first biological signals collected a plurality of times.

That is, the first sleep-aiding audio signal may be played a plurality of times, and the first biological signals may be collected when the first sleep-aiding audio signal is played; and then, the sleep quality of the first user is determined based on the first biological signals collected a plurality of times.

For example, whole sleep qualities of the first user in the plurality of times may be obtained based on the first biological signals collected a plurality of times, and a whole sleep quality that is of the first user and that is obtained after statistics collection may be determined based on the whole sleep qualities of the first user in the plurality of times.

For example, if the first sleep-aiding audio signal is played in 10 sleep cycles, that is, played 10 times, an average value of whole sleep qualities that are of the first user in the 10 times and that are obtained after the first sleep-aiding audio signal is played in the 10 sleep cycles is calculated, and the average value is used as a whole sleep quality that is of the first user and that is obtained after statistics collection. The whole sleep quality of the first user may be obtained through calculation by using the foregoing linear sleep evaluation method, or may be obtained through calculation by using the foregoing weighted sleep evaluation method.

For example, fragmented sleep qualities of the first user in the plurality of times may be obtained based on the first biological signals collected a plurality of times, and a fragmented sleep quality that is of the first user and that is obtained after statistics collection may be determined based on the fragmented sleep qualities of the first user in the plurality of times.

For example, if the first sleep-aiding audio signal is played in 10 sleep cycles, that is, played for 10 times, sleep qualities corresponding to the first sleep stage of the first user in the 10 times may be obtained, an average value of the sleep qualities corresponding to the first sleep stage of the first user in the 10 times may be calculated, and the average value may be used as a sleep quality that corresponds to the first sleep stage of the first user and that is obtained after statistics collection. It should be noted that the playing in the 10 sleep cycles does not mean that the first sleep-aiding audio signal is played in all stages of the 10 sleep cycles, and may be alternatively played only in N1 stages of the sleep cycles.

In this way, the fragmented sleep quality corresponding to the sleep-aiding audio signal may be continuously updated, so that accuracy of evaluating a sleep-aiding effect of the sleep-aiding audio signal can be improved.

Further, statistics collection may be performed on a whole sleep quality evaluation result of the user based on long-term data, to obtain a whole sleep quality obtained after the statistics collection.

In this way, impact of the sleep-aiding audio signal on the sleep quality of the user can be evaluated based on long-term collected data, so that a sleep-aiding effect of the sleep-aiding audio signal can be more accurately reflected.

S530: Update the sleep-aiding audio library based on the sleep quality of the first user.

In this embodiment of this application, the updating the sleep-aiding audio library is updating the sleep-aiding audio signals in the sleep-aiding audio library.

Optionally, the updating the sleep-aiding audio library based on the sleep quality of the first user includes: updating a sequence of the sleep-aiding audio signals in the sleep-aiding audio library based on the sleep quality of the first user; and/or deleting one or more sleep-aiding audio signals from the sleep-aiding audio library based on the sleep quality of the first user.

For example, this step may be performed by the audio sorting module 460 in FIG. 4.

For example, the sleep-aiding audio signals in the sleep-aiding audio library may be sorted by using a method such as bubble sort, selection sort, insertion sort, merge sort, or quick sort. Alternatively, a sorting result may be obtained by using a neural network model. A specific sorting form is not limited in this embodiment of this application.

Optionally, step S530 may include: determining a score of the first sleep-aiding audio signal based on the sleep quality of the first user, and updating the sleep-aiding audio library based on the score. This step may be performed by the audio evaluation module 450 and the audio sorting module 460 in FIG. 4.

For example, a mapping relationship may be established between the first sleep-aiding audio signal and the sleep quality that is of the first user and that corresponds to the first sleep-aiding audio signal, and the sleep quality of the first user may be used as the score of the first sleep-aiding audio signal. The sleep quality corresponding to the first sleep-aiding audio signal is a sleep quality that is of the first user and that is determined by using the bioelectrical signal collected when the first sleep-aiding audio signal is played.

For another example, the first sleep-aiding audio signal may be scored based on the sleep quality of the first user.

For another example, the first sleep-aiding audio signal may be scored based on a user preference and the sleep quality of the first user. For example, the first sleep-aiding audio signal is scored based on a quantity of times the first user selects the first sleep-aiding audio signal and the sleep quality of the first user. Alternatively, the first sleep-aiding audio signal may be scored based on the sleep quality of the first user, and the plurality of sleep-aiding audio signals in the sleep-aiding audio library may be sorted based on the scoring, to obtain a first sorting result; and the first sorting result may be adjusted based on the user preference, to obtain a second sorting result, and the sleep-aiding audio library may be updated based on the sorting result.

For ease of explanation and description, in this embodiment of this application, the sleep-aiding audio signals are sorted in descending order of sleep qualities of the user.

Optionally, the sleep-aiding audio library may include audio signals corresponding to whole sleep and/or audio signals corresponding to the plurality of sleep stages.

It should be noted that the plurality of sleep-aiding audio signals corresponding to the whole sleep may be the same as or different from the plurality of sleep-aiding audio signals corresponding to the plurality of sleep stages.

As described above, the sleep quality of the first user may include the whole sleep quality of the first user and/or the fragmented sleep quality of the first user.

The updating a sequence of the sleep-aiding audio signals in the sleep-aiding audio library based on the sleep quality of the first user may include: updating, based on the whole sleep quality of the first user, a sequence of the plurality of sleep-aiding audio signals corresponding to the whole sleep; or updating, based on the sleep quality corresponding to the at least one sleep stage of the first user, a sequence of a plurality of sleep-aiding audio signals corresponding to the at least one sleep stage.

The deleting one or more sleep-aiding audio signals from the sleep-aiding audio library based on the sleep quality of the first user may include: deleting, based on the whole sleep quality of the first user, one or more sleep-aiding audio signals from the plurality of sleep-aiding audio signals corresponding to the whole sleep; or deleting, based on the sleep quality corresponding to the at least one sleep stage of the first user, one or more sleep-aiding audio signals from the plurality of sleep-aiding audio signals corresponding to the at least one sleep stage. For example, a sleep-aiding audio signal ranked low may be deleted.

The first sleep-aiding audio signal may be one of the plurality of sleep-aiding audio signals corresponding to the whole sleep, or may be one of the plurality of sleep-aiding audio signals corresponding to the plurality of sleep stages.

Alternatively, the first sleep-aiding audio signal may be a newly added sleep-aiding audio signal.

The newly added sleep-aiding audio signal is a sleep-aiding audio signal that has no corresponding sleep quality of the first user. For example, the newly added sleep-aiding audio signal may be a sleep-aiding audio signal uploaded to the sleep-aiding audio library for the first time. The newly added sleep-aiding audio signal may be a sleep-aiding audio signal added by a system, or may be a sleep-aiding audio signal added by the user.

For example, the first sleep-aiding audio signal may be one of the plurality of sleep-aiding audio signals corresponding to the whole sleep. The whole sleep quality of the first user is obtained according to step S520; and then, the sequence of the plurality of sleep-aiding audio signals corresponding to the whole sleep may be updated based on the whole sleep quality.

For example, the first sleep-aiding audio signal may be a newly added sleep-aiding audio signal. The whole sleep quality of the first user may be obtained according to step S520; and then, the plurality of sleep-aiding audio signals corresponding to the whole sleep or the sequence of the plurality of sleep-aiding audio signals corresponding to the whole sleep may be updated based on the whole sleep quality.

For example, a lower sleep quality score indicates a better sleep quality of the user. In this case, when a whole sleep quality score of the first user is less than or equal to a first threshold, the first sleep-aiding audio signal is added to the plurality of sleep-aiding audio signals corresponding to the whole sleep, that is, the plurality of sleep-aiding audio signals corresponding to the whole sleep are updated. When a whole sleep quality score of the first user is greater than the first threshold, the first sleep-aiding audio signal is deleted.

For another example, the first sleep-aiding audio signal is added to the plurality of sleep-aiding audio signals corresponding to the whole sleep, and the sequence of the plurality of sleep-aiding audio signals corresponding to the whole sleep is updated based on the whole sleep quality of the first user. Further, a sleep-aiding audio signal ranked last may be deleted. It may also be understood that the plurality of sleep-aiding audio signals corresponding to the whole sleep are updated.

For example, the first sleep-aiding audio signal may be one of the plurality of sleep-aiding audio signals corresponding to the whole sleep, and the first sleep-aiding audio signal does not belong to a plurality of sleep-aiding audio signals corresponding to the first sleep stage. That is, the first sleep-aiding audio signal is a newly added sleep-aiding audio signal relative to the plurality of sleep-aiding audio signals corresponding to the first sleep stage. The sleep quality corresponding to the first sleep stage of the first user may be obtained according to step S520; and then the plurality of sleep-aiding audio signals corresponding to the first sleep stage or a sequence of the plurality of sleep-aiding audio signals corresponding to the first sleep stage may be updated based on the sleep quality corresponding to the first sleep stage.

Optionally, the method 500 further includes step S540.

S540: Play a target sleep-aiding audio signal.

For example, step S540 may be performed by the audio play module 420 in FIG. 4.

Specifically, the target sleep-aiding audio signal may be determined based on an updated sleep-aiding audio library. For example, the target sleep-aiding audio signal may be a sleep-aiding audio signal ranked first in a plurality of sleep-aiding audio signals in the sleep-aiding audio library. For another example, a sequence of the plurality of sleep-aiding audio signals may be displayed to the first user, so that the first user can select a sleep-aiding audio signal based on the sequence.

Optionally, the method 500 further includes: determining the target sleep-aiding audio signal based on the updated sleep-aiding audio library.

For example, the target sleep-aiding audio signal may be determined based on updated sleep-aiding audio signals corresponding to the whole sleep.

For example, the target sleep-aiding audio signal may be determined based on updated sleep-aiding audio signals corresponding to the at least one sleep stage. In this case, the target sleep-aiding audio signal is used to be played for the first user when the first user is in the at least one sleep stage. That is, when the first user is in different sleep stages, corresponding target sleep-aiding audio signals may be played based on updated sleep-aiding audio signals corresponding to the different sleep stages. Different sleep stages may correspond to a same target sleep-aiding audio signal or different target sleep-aiding audio signals.

According to the solution in this embodiment of this application, the sleep-aiding audio signal may be updated based on the sleep quality of the user. That is, information related to sleep of the user is determined by using the biological signal; and then, the sleep quality of the user is evaluated, and a sleep-aiding effect of the sleep-aiding audio signal is evaluated based on the sleep quality of the user. Compared with updating an audio signal based on another parameter, the solution in this application can better meet a sleep quality requirement of the user and improve a sleep-aiding effect.

In addition, an evaluation that is of a sleep quality of a user and that is obtained by using a same sleep-aiding audio signal may be continuously updated, so that accuracy of evaluating a sleep-aiding effect of the sleep-aiding audio signal can be improved.

Further, the method 500 further includes step S550. Step S550 is an optional step.

S550: Determine a sleep quality of a second user.

In this case, step S530 may include: updating the sleep-aiding audio library based on the sleep quality of the first user and the sleep quality of the second user.

The sleep quality of the second user is determined based on a second biological signal, the second biological signal is a biological signal of the second user, and the second biological signal is collected when a second sleep-aiding audio signal in the sleep-aiding audio library is played. The first sleep-aiding audio signal and the second sleep-aiding audio signal may be a same sleep-aiding audio signal, or may be different sleep-aiding audio signals.

That is, the sleep-aiding audio library may be determined based on sleep qualities of a plurality of users.

Specifically, the sleep quality of the second user may be determined with reference to step S510 and step S520.

For example, the determining sleep quality of a second user may alternatively include: receiving the sleep quality of the second user from another device.

For example, the sleep-aiding audio library may include a plurality of sleep-aiding audio signals corresponding to a group user.

The updating the sleep-aiding audio library based on the sleep quality of the first user and the sleep quality of the second user may be updating, based on the sleep quality of the first user and the sleep quality of the second user, the plurality of sleep-aiding audio signals corresponding to the group user.

For example, if the first sleep-aiding audio signal and the second sleep-aiding audio signal are a same sleep-aiding audio signal, a sleep quality of the group user may be determined based on the sleep quality of the first user and the sleep quality of the second user; and then, the plurality of sleep-aiding audio signals corresponding to the group user may be updated based on the sleep quality of the group user.

Specifically, an average value of the sleep quality of the first user and the sleep quality of the second user may be calculated, and the average value may be used as the sleep quality of the group user. Herein, only the first user and the second user are used as an example to describe a method for determining the sleep quality of the group user. A quantity of users is not limited in this embodiment of this application.

The sleep quality of the first user and the sleep quality of the second user each may be determined based on a biological signal collected once, or may be determined based on biological signals collected a plurality of times.

The sleep quality of the group user may include a whole sleep quality of the group user and a fragmented sleep quality of the group user. The plurality of sleep-aiding audio signals corresponding to the group user may include a plurality of sleep-aiding audio signals corresponding to whole sleep of the group user and a plurality of sleep-aiding audio signals corresponding to a plurality of sleep stages of the group user. The plurality of sleep-aiding audio signals corresponding to the whole sleep of the group user may be determined based on the whole sleep quality of the group user. The plurality of sleep-aiding audio signals corresponding to the plurality of sleep stages of the group user may be determined based on the fragmented sleep quality of the group user.

For example, an average value of the sleep quality corresponding to the first sleep stage of the first user and a sleep quality corresponding to a first sleep stage of the second user may be calculated, and the average value may be used as the sleep quality corresponding to a first sleep stage of the group user. Then, a plurality of sleep-aiding audio signals corresponding to the first sleep stage of the group user may be updated based on the sleep quality corresponding to the first sleep stage of the group user.

Herein, only the two users are used as an example to describe a method for determining the fragmented sleep quality of the group user, and a quantity of users may be another quantity. For example, a same sleep-aiding audio signal is played for m users to obtain sleep qualities corresponding to first sleep stages of the m users, an average value of the sleep qualities corresponding to the m first sleep stages may be calculated, and the average value may be used as the sleep quality corresponding to the first sleep stage of the group users.

For example, the m users may be users with a same feature. For example, the m users may be users of a same age stage. Alternatively, the m users may be users of a same gender. Alternatively, the m users may be users of a same area.

For another example, an average value of the whole sleep quality of the first user and a whole sleep quality of the second user may be calculated, and the average value may be used as the whole sleep quality of the group user. Then, a plurality of sleep-aiding audio signals corresponding to the whole sleep quality of the group user may be updated based on the whole sleep quality of the group user. The whole sleep quality of the first user and the whole sleep quality of the second user may be determined based on the foregoing linear sleep evaluation method, that is, may be whole linear sleep qualities. In this case, it may be considered that the whole sleep quality of the group user may be determined based on the foregoing linear sleep evaluation method, that is, may be a whole linear sleep quality of the group user. Alternatively, the whole sleep quality of the first user and the whole sleep quality of the second user may be determined based on the foregoing weighted sleep evaluation method, that is, may be whole weighted sleep qualities. In this case, it may be considered that the whole sleep quality of the group user may be determined based on the foregoing weighted sleep evaluation method, that is, may be a whole weighted sleep quality of the group user.

Herein, only the two users are used as an example to describe a method for determining the whole sleep quality of the group user, and a quantity of users may be another quantity. For example, a same sleep-aiding audio signal is played for m users to obtain whole sleep qualities of the m users, an average value of the m whole sleep qualities is calculated, and the average value is used as the whole sleep quality of the group user.

The whole sleep qualities of the m users may be obtained through calculation by using the foregoing linear sleep evaluation method, or may be obtained through calculation by using the foregoing weighted sleep evaluation method.

For example, the m users may be users with a same feature. For example, the m users may be users of a same age stage. Alternatively, the m users may be users of a same gender. Alternatively, the m users may be users of a same area.

For example, the sleep-aiding audio library may include a plurality of sleep-aiding audio signals corresponding to the first user and a plurality of sleep-aiding audio signals corresponding to the second user.

The updating the sleep-aiding audio library based on the sleep quality of the first user and the sleep quality of the second user may be updating, based on the sleep quality of the first user, the plurality of sleep-aiding audio signals corresponding to the first user, and updating, based on the sleep quality of the second user, the plurality of sleep-aiding audio signals corresponding to the second user.

The following uses examples (a manner 1 and a manner 2) to describe a specific implementation of step S540 when the method 500 includes step S550.

Manner 1:

If the first user plays a sleep-aiding audio signal in the sleep-aiding audio library for the first time, a target sleep-aiding audio signal may be played based on the plurality of sleep-aiding audio signals corresponding to the group user.

For example, if the first user plays a sleep-aiding audio signal in the sleep-aiding audio library for the first time, a target sleep-aiding audio signal may be played based on the plurality of sleep-aiding audio signals corresponding to the whole sleep of the group user. The plurality of sleep-aiding audio signals corresponding to the whole sleep of the group user may be determined based on the whole sleep quality of the group user.

The whole sleep quality of the group user may be determined based on the linear sleep evaluation method. Alternatively, the whole sleep quality of the group user may be determined based on the weighted sleep evaluation method.

For another example, if the first user plays a sleep-aiding audio signal in the sleep-aiding audio library for the first time, target sleep-aiding audio signals corresponding to different sleep stages may be played based on a plurality of sleep-aiding audio signals corresponding to the different sleep stages of the group user when the user is in the different sleep stages.

Specifically, when the user is in the W period, a sleep-aiding audio signal ranked first in a plurality of sleep-aiding audio signals corresponding to a W period of the group user is played; when the user is in the N1 period, a sleep-aiding audio signal ranked first in a plurality of sleep-aiding audio signals corresponding to an N1 period of the group user is played; when the user is in the N2 period, a sleep-aiding audio signal ranked first in a plurality of sleep-aiding audio signals corresponding to an N2 period of the group user is played; when the user is in the N3 period, a sleep-aiding audio signal ranked first in a plurality of sleep-aiding audio signals corresponding to an N3 period of the group user is played; when the user is in the N4 period, a sleep-aiding audio signal ranked first in a plurality of sleep-aiding audio signals corresponding to an N4 period of the group user is played; or when the user is in the REM period, a sleep-aiding audio signal ranked first in a plurality of sleep-aiding audio signals corresponding to an REM period of the group user is played.

Manner 2:

If the first user does not play a sleep-aiding audio signal in the sleep-aiding audio library for the first time, a target sleep-aiding audio signal may be played based on the plurality of sleep-aiding audio signals corresponding to the first user.

For example, if the first user does not play a sleep-aiding audio signal in the sleep-aiding audio library for the first time, a target sleep-aiding audio signal may be played based on the plurality of sleep-aiding audio signals corresponding to the whole sleep of the first user. The plurality of sleep-aiding audio signals corresponding to the whole sleep of the first user may be determined based on the whole sleep quality of the first user.

The whole sleep quality of the first user may be determined based on the linear sleep evaluation method. Alternatively, the whole sleep quality of the first user may be determined based on the weighted sleep evaluation method.

For another example, if the first user does not play a sleep-aiding audio signal in the sleep-aiding audio library for the first time, target sleep-aiding audio signals corresponding to different sleep stages may be played based on a plurality of sleep-aiding audio signals corresponding to the different sleep stages of the first user when the first user is in the different sleep stages.

In this way, the sleep-aiding audio library may be updated based on sleep qualities of a plurality of users, so that suitable groups of the sleep-aiding audio library are increased. That is, the sleep-aiding audio library is applicable to more users. Especially, when a user uses the sleep-aiding audio library for the first time, a sleep-aiding audio signal is played in the sleep-aiding audio library determined based on the sleep qualities of the plurality of users, so that a relatively good sleep-aiding effect can still be ensured when there is no related data of the user.

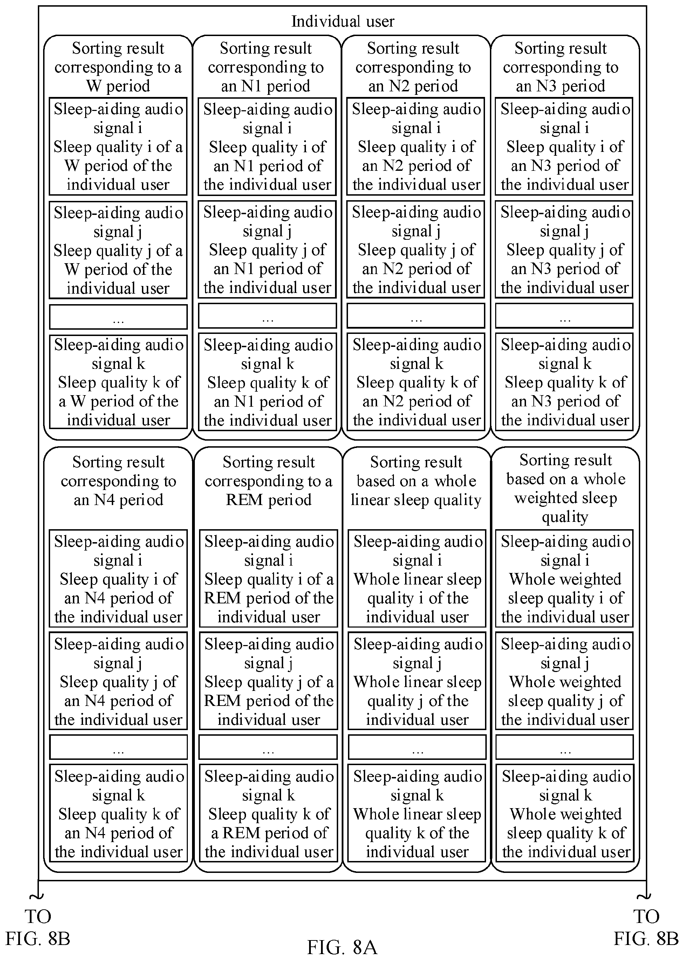

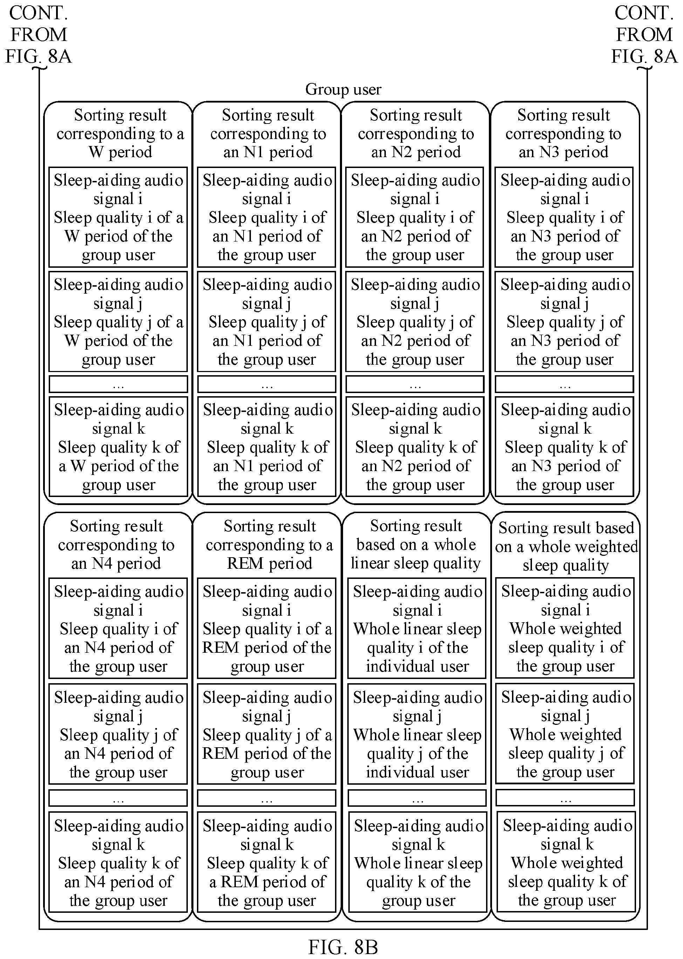

For example, the target sleep-aiding audio signal in the foregoing manners may be a sleep-aiding audio signal ranked first in the plurality of sleep-aiding audio signals in the sleep-aiding audio library. Alternatively, the target sleep-aiding audio signal in the foregoing manners may be selected by the user. For example, a sequence of the plurality of sleep-aiding audio signals may be displayed to the first user, so that the first user can select a sleep-aiding audio signal based on the sequence. For example, all or some sorting results in FIG. 8A and FIG. 8B may be displayed to the user.

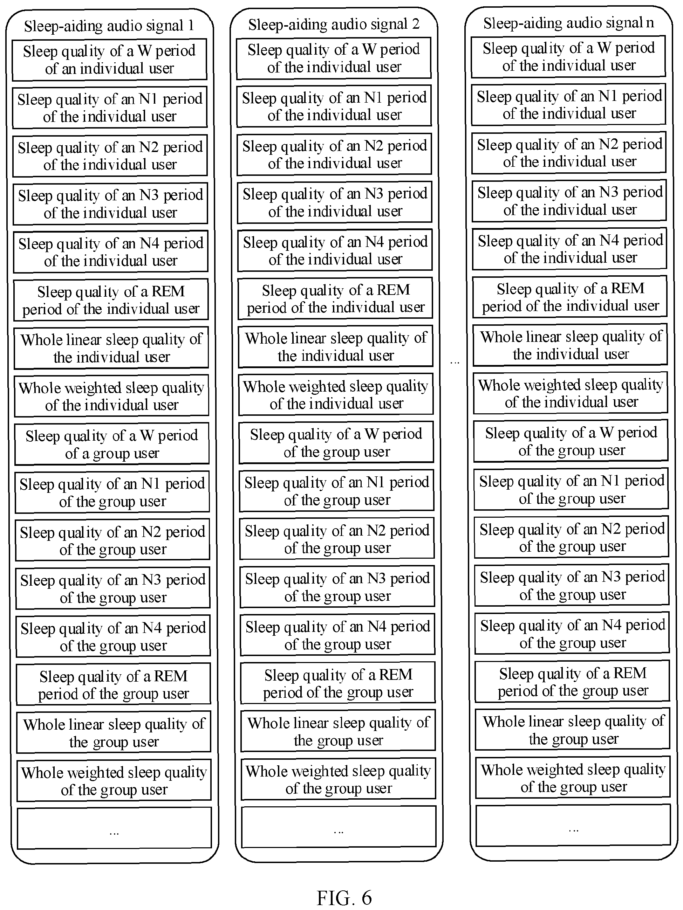

The following describes, with reference to FIG. 6 to FIG. 8B, a method for updating the sequence of the plurality of sleep-aiding audio signals in the sleep-aiding audio library based on the sleep quality. The method may be considered as an implementation of step S530. The method includes step 1 to step 3, and the following describes step 1 to step 3.

Step 1: Establish mapping relationships between sleep-aiding audio signals and sleep qualities corresponding to the sleep-aiding audio signals.

For example, this step may be performed by the audio evaluation module 450 in FIG. 4.

A manner of establishing the mapping relationships between the sleep-aiding audio signals and the sleep qualities is described by using a sleep-aiding audio signal 1 as an example.

For the sleep-aiding audio signal 1, a sleep quality corresponding to the sleep-aiding audio signal 1 may be obtained in step S520. A mapping relationship is established between the sleep quality and the sleep-aiding audio signal 1.

The sleep quality may include one or more of the following: a sleep quality of a W period of an individual user, a sleep quality of an N1 period of the individual user, a sleep quality of an N2 period of the individual user, a sleep quality of an N3 period of the individual user, a sleep quality of an N4 period of the individual user, a sleep quality of a REM period of the individual user, a whole linear sleep quality of the individual user, a whole weighted sleep quality of the individual user, a sleep quality of a W period of a group user, a sleep quality of an N1 period of the group user, a sleep quality of an N2 period of the group user, a sleep quality of an N3 period of the group user, a sleep quality of an N4 period of the group user, a sleep quality of a REM period of the group user, a whole linear sleep quality of the group user, and a whole weighted sleep quality of the group user.

The individual user may include one user, or may include a plurality of users. The whole linear sleep quality is a whole sleep quality determined by using the foregoing linear sleep evaluation method. The whole weighted sleep quality is a whole sleep quality determined by using the foregoing weighted sleep evaluation method.

FIG. 6 shows mapping relationships between n sleep-aiding audio signals and sleep qualities according to an embodiment of this application. It should be noted that the mapping relationships in FIG. 6 are merely an example. Sleep qualities for establishing mapping relationships with sleep-aiding audio signals may include only some sleep qualities in FIG. 6, or may include other sleep qualities corresponding to the sleep-aiding audio signals. The n sleep-aiding audio signals may correspond to a same quantity of sleep qualities or different quantities of sleep qualities, and n is a positive integer.

Further, the sleep-aiding audio signals may be scored based on the sleep qualities corresponding to the sleep-aiding audio signals, to obtain scores of the sleep-aiding audio signals under different sleep qualities; and mapping relationships are established between the sleep-aiding audio signals and the scores.

Step 2: Integrate sleep-aiding audio signals corresponding to different sleep stages.

For example, this step may be performed by the audio evaluation module 450 in FIG. 4.

For example, a plurality of sleep-aiding audio signals corresponding to at least one sleep stage of an individual user may be integrated, or a plurality of sleep-aiding audio signals corresponding to whole sleep of the individual user may be integrated.

For example, a sleep quality 1 that is of an N1 period of the individual user and that corresponds to the sleep-aiding audio signal 1 is determined based on the mapping relationship that is between the sleep-aiding audio signal 1 and the sleep quality and that is obtained in step 1, and a sleep quality 1 that is of the N1 period of the individual user and that corresponds to a sleep-aiding audio signal 2 is determined based on a mapping relationship that is between the sleep-aiding audio signal 2 and a sleep quality and that is obtained in step 1. In this case, sleep-aiding audio signals corresponding to the N1 period of the individual user include the sleep-aiding audio signal 1 and the sleep-aiding audio signal 2.

The plurality of sleep-aiding audio signals corresponding to the whole sleep of the individual user may include a plurality of sleep-aiding audio signals integrated based on a whole linear sleep quality of the individual user, or may include a plurality of sleep-aiding audio signals integrated based on a whole weighted sleep quality of the individual user.

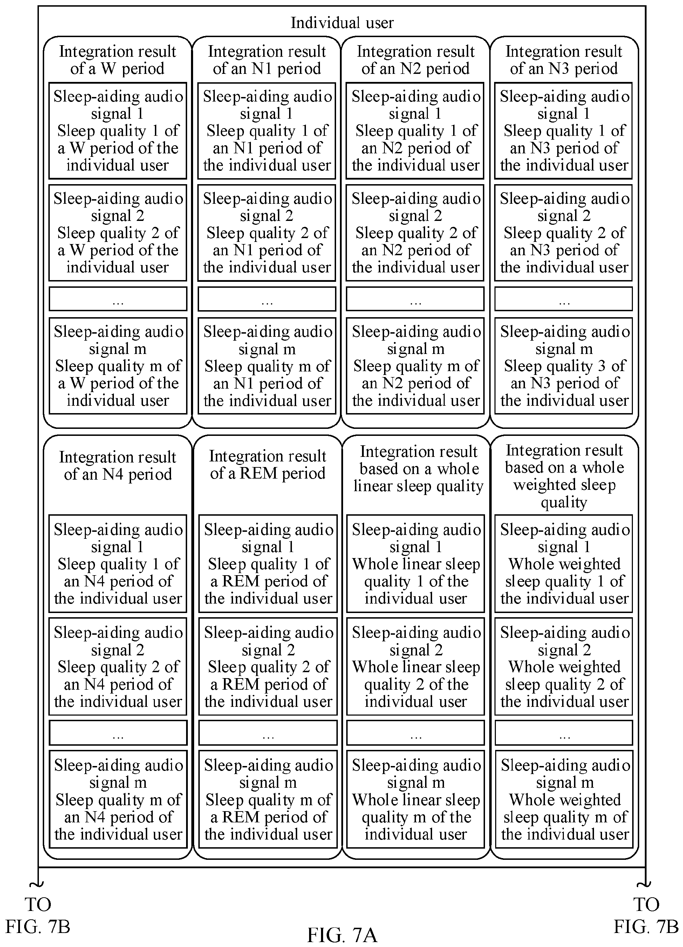

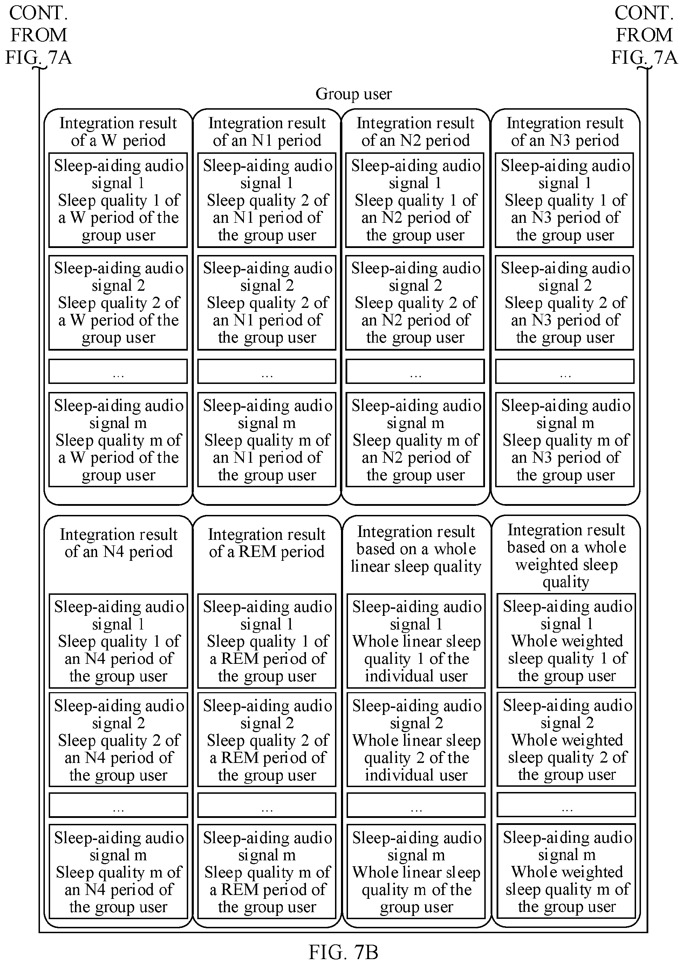

FIG. 7A and FIG. 7B show an integration result of different sleep-aiding audio signals according to an embodiment of this application. As shown in FIG. 7A and FIG. 7B, for an individual user, an integration result of a W period includes m sleep-aiding audio signals corresponding to sleep qualities of m W periods, or sleep qualities of m W periods corresponding to m sleep-aiding audio signals. m is a positive integer, and m may be the same as or different from n. It should be noted that, in FIG. 7A and FIG. 7B, only an example in which each sleep stage corresponds to m sleep-aiding audio signals is used. A quantity of sleep-aiding audio signals corresponding to each sleep stage is not limited in this embodiment of this application. All sleep stages may correspond to a same quantity of sleep-aiding audio signals or different quantities of sleep-aiding audio signals.

It should be noted that, in FIG. 7A and FIG. 7B, an integration result of only one individual user is used as an example. A quantity of individual users is not limited in this embodiment of this application.

For example, a plurality of sleep-aiding audio signals corresponding to at least one sleep stage of a group user may be integrated, or a plurality of sleep-aiding audio signals corresponding to whole sleep of the group user may be integrated.

For example, a sleep quality 1 that corresponds to an N1 period of the group user and that corresponds to the sleep-aiding audio signal 1 is determined based on the mapping relationship that is between the sleep-aiding audio signal 1 and the sleep quality and that is obtained in step 1, and a sleep quality 2 that corresponds to the N1 period of the group user and that corresponds to a sleep-aiding audio signal 2 is determined based on a mapping relationship that is between the sleep-aiding audio signal 2 and a sleep quality and that is obtained in step 1. In this case, sleep-aiding audio signals corresponding to the N1 period of the group user include the sleep-aiding audio signal 1 and the sleep-aiding audio signal 2.

The plurality of sleep-aiding audio signals corresponding to the whole sleep of the group user may include a plurality of sleep-aiding audio signals integrated based on a whole linear sleep quality of the group user, or may include a plurality of sleep-aiding audio signals integrated based on a whole weighted sleep quality of the group user.

Step 3: Update the sleep-aiding audio library based on an integration result.

Specifically, a plurality of sleep-aiding audio signals corresponding to different sleep stages in the sleep-aiding audio library may be sorted based on integration results of the different sleep stages. For example, the sleep-aiding audio signals may be sorted by using a method such as bubble sort, selection sort, insertion sort, merge sort, or quick sort. A specific sorting form is not limited in this embodiment of this application.

For example, this step may be performed by the audio sorting module 460 in FIG. 4.

FIG. 8A and FIG. 8B are a schematic diagram of a sleep-aiding audio signal sorting result according to an embodiment of this application. As shown in FIG. 8A and FIG. 8B, a plurality of sleep-aiding audio signals corresponding to different sleep stages may be sorted to obtain sorting results corresponding to the different sleep stages. In this way, a sorting result may be selected based on a requirement, and then a suitable sleep-aiding audio signal may be played, to meet requirements of different users. FIG. 8A and FIG. 8B show only an example in which a sorting result is a sleep-aiding audio signal i, a sleep-aiding audio signal j, ..., and a sleep-aiding audio signal k. This does not mean that all sleep stages correspond to a same sorting result.

It should be noted that the example descriptions are merely intended to help a person skilled in the art understand this embodiment of this application, instead of limiting this embodiment of this application to the illustrated specific value or specific scenario. A person skilled in the art clearly can make various equivalent modifications or changes according to the examples described above, and such modifications or changes also fall within the scope of embodiments of this application.

The following describes apparatus embodiments in the embodiments of this application in detail with reference to the accompanying drawings. It should be understood that an apparatus described in the following can perform the method in the foregoing embodiments of this application. To avoid unnecessary repetition, the following appropriately omits repeated descriptions when introducing the apparatus in the embodiments of this application.

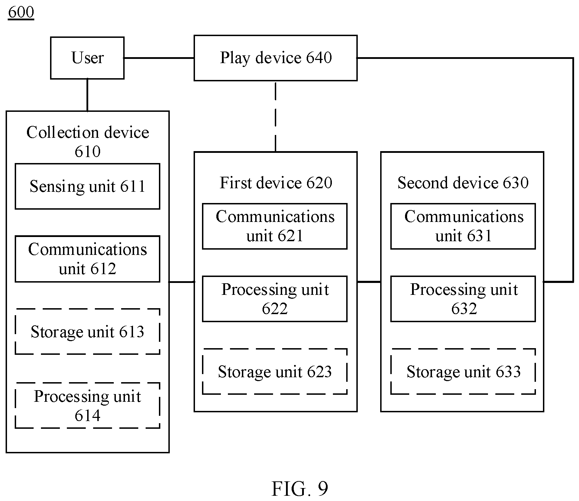

FIG. 9 shows a sleep-aiding audio signal updating system 600 according to an embodiment of this application. For example, the system 600 shown in FIG. 9 may be configured to complete functions that need to be performed by the modules in FIG. 4, or the system 600 may be configured to perform the method 500 in FIG. 5. The system 600 may include a collection device 610, a first device 620, a second device 630, and a play device 640.

For example, the first device 620 may be a terminal device or a cloud server, and the second device 630 may be a cloud server or a terminal device.

For example, the first device 620 is a terminal device, and the second device 630 is a cloud server.

For another example, the first device 620 is a terminal device, and the second device 630 is another terminal device.

The collection device 610 may include a sensing unit 611 and a communications unit 612.

The sensing unit 611 may obtain a bioelectrical signal collected by using a multi-modal sensor device.

The communications unit 612 may be configured to transmit a first biological signal to the first device 620. In this case, the bioelectrical signal is the first biological signal.

Optionally, the collection device 610 may further include a storage unit 613, and the storage unit 613 may be configured to store the bioelectrical signal collected by the sensing unit 611.

Optionally, the collection device 610 may further include a processing unit 614, and the processing unit 614 may be configured to preprocess the bioelectrical signal, for example, filter the bioelectrical signal, to obtain a processed bioelectrical signal. In this case, the preprocessed bioelectrical signal is the first biological signal, that is, the communications unit 612 transmits the preprocessed bioelectrical signal to the first device 620. The storage unit 613 may be configured to store the preprocessed bioelectrical signal.

The first device 620 may include a communications unit 621 and a processing unit 622.

The communications unit 621 may be configured to perform data transmission with the collection device 610 and the second device 630. Specifically, the communications unit 621 may be configured to: receive data sent by the communications unit 612, and send data obtained after the processing unit 622 performs processing to a communications unit 631.

The processing unit 622 may be configured to process the data received by the communications unit 621. For example, the processing unit 622 may be configured to perform step S510 in FIG. 5, the processing unit 622 may be configured to perform step S510 and step S520 in FIG. 5, or the processing unit 622 may be configured to perform step S510 to step S530 in FIG. 5. It should be noted that, when the method 500 does not include step S550, if the processing unit 622 performs step S510 to step S530 in FIG. 5, the system 600 may not include the second device 630. For a structure of the system 600, refer to a system 700.

Optionally, the first device 620 further includes a storage unit 623. The storage unit 623 may be configured to store the data received by the communications unit 621 or the data obtained after the processing unit 622 performs processing.

The second device 630 may include the communications unit 631 and a processing unit 632.

The communications unit 631 may be configured to communicate with the first device 620, to implement information exchange between the first device 620 and the second device 630. Specifically, the second device 630 may obtain, by using the communications unit 631, data sent by the first device 620, and feed back a result obtained after the processing unit 632 performs processing to the first device 620 or the play device 640 by using the communications unit 631.

The processing unit 632 may be configured to process the data received by the communications unit 631, to obtain the processed result.

For example, the processing unit 632 may be configured to perform step S530 in FIG. 5, the processing unit 632 may be configured to perform step S520 and step S530 in FIG. 5, or the processing unit 632 may be configured to perform step S550 in FIG. 5.

Optionally, the second device 630 further includes a storage unit 633. The storage unit 633 may be configured to store the data received by the communications unit 631 or the data obtained after the processing unit 632 performs processing. For example, the storage unit 633 may be configured to store a sleep-aiding audio library.

The play device 640 may be configured to play a sleep-aiding audio signal.

Optionally, the collection device 610 may be disposed in the first device 620. Alternatively, it may be understood that the first device 620 includes a collection unit, so that a corresponding function of the collection device 610 can be implemented. For example, the first device 620 may be a head-mounted device. For example, the head-mounted device may include a head cover, an eye cover, a headset, or a pillow.

Optionally, the play device 640 may be disposed in the first device 620. Alternatively, it may be understood that the first device 620 includes a play unit, so that a corresponding function of the play device 640 can be implemented. For example, the first device 620 may be a device such as a wearable device, a mobile terminal, or a sound box. For example, the wearable device may include a headset. For another example, the mobile terminal may include a mobile phone or a tablet computer.

That is, the collection device 610 or the play device 640 may not be an independent device, but may implement a corresponding function in a form of a unit integrated into the first device 620. Alternatively, it may be understood that the system 600 includes the first device 620 and the second device 630. The first device 620 may be further configured to perform an operation of the collection device 610, to implement a corresponding function. Alternatively, the first device 620 may be further configured to perform an operation of the play device 640, to implement a corresponding function.

The following uses an example to describe a process of updating a sleep-aiding audio signal by using the system shown in FIG. 9.

Step 1: The first device 620 obtains a first biological signal collected when a first audio signal in a sleep-aiding audio library is played.

The first biological signal is a biological signal corresponding to a first user. Specifically, the first biological signal may be a bioelectrical signal of the user, or may be a preprocessed bioelectrical signal.

For example, the processing unit 622 may obtain the first biological signal that is sent by the collection device 610 and that is received by the communications unit 621.

For example, the first device 620 may include a collection unit, and the collection unit is configured to collect the bioelectrical signal of the user. That is, the first device 620 may not include the communications unit 621, and the processing unit 622 may obtain the bioelectrical signal collected by the collection unit.

Further, the collection unit may preprocess the bioelectrical signal, for example, filter the bioelectrical signal.

Step 2: The first device 620 determines a sleep quality of the first user based on the first biological signal. For specific descriptions, refer to step S520 in the method 500. Details are not described herein again.

Step 3: The second device 630 obtains the sleep quality of the first user, and updates the sleep-aiding audio library based on the sleep quality of the first user. For specific descriptions, refer to step S530 in the method 500. Details are not described herein again.

The first device 620 may send the sleep quality of the first user to the second device 630 by using the communications unit 621. The processing unit 632 in the second device may update the sleep-aiding audio library based on the sleep quality of the first user.

Optionally, the second device 630 may obtain sleep qualities of a plurality of users, and update the sleep-aiding audio library based on the sleep qualities of the plurality of users. For example, the second device 630 may be a cloud server, and the first device 620 may be a terminal device. The second device 630 may obtain sleep qualities that are of a plurality of users and that are sent by a plurality of terminal devices, and update the sleep-aiding audio library based on the sleep qualities of the plurality of users. For specific descriptions, refer to step S550 in the method 500. Details are not described herein again.

Step 4: The play device 640 plays a target sleep-aiding audio signal. This step is an optional step.

For example, the target sleep-aiding audio signal may be determined based on an updated sleep-aiding audio library. For example, the target sleep-aiding audio signal may be a sleep-aiding audio signal ranked first in a plurality of sleep-aiding audio signals in the sleep-aiding audio library. For another example, a sequence of the plurality of sleep-aiding audio signals may be displayed to the first user, so that the first user can select a sleep-aiding audio signal based on the sequence.

For example, the play device 640 obtains the updated sleep-aiding audio library, and then plays the target sleep-aiding audio signal.

For example, the play device 640 obtains the target sleep-aiding audio signal, and then plays the target sleep-aiding audio signal. For example, the second device 630 may determine the target sleep-aiding audio signal and indicate the play device 640 to play the target sleep-aiding audio signal. Alternatively, the first device 620 obtains the target sleep-aiding audio signal and indicates the play device 640 to play the target sleep-aiding audio signal. Alternatively, the first device 620 obtains the updated sleep-aiding audio library, determines the target sleep-aiding audio signal, and indicates the play device 640 to play the target sleep-aiding audio signal.

For example, the first device 620 may include a play unit, and the play unit is configured to play an audio signal. That is, the first device 620 may be configured to play the target sleep-aiding audio signal. For example, the second device 630 may determine the target sleep-aiding audio signal and indicate the first device 620 to play the target sleep-aiding audio signal. Alternatively, the first device 620 obtains the target sleep-aiding audio signal and plays the target sleep-aiding audio signal.

For specific descriptions, refer to the manner 1 and the manner 2 in the foregoing method 500. Details are not described herein again.

It should be understood that the foregoing process is merely an example. In this embodiment of this application, the devices in the system 600 may cooperate to perform the sleep-aiding audio signal updating method in the embodiments of this application. Operations performed by the devices in the system 600 may be the same as or different from those in the foregoing process. This is not limited in this embodiment of this application.

For example, in step 2, the first device 620 may send data related to the first biological signal to the second device 630, and the second device 630 determines the sleep quality of the first user, that is, the second device 630 performs step S520 in the method 500. The data related to the first biological signal may be the first biological signal, or may be a result obtained after the first biological signal is processed. For example, the data related to the first biological signal may be the foregoing first processed signals. For another example, the data related to the first biological signal may be a sleep period division result determined based on the first biological signal.

For example, in step 3, the first device 620 may update the sleep-aiding audio library based on the sleep quality of the first user, and the second device 630 may receive a sleep quality that is of a second user and that is sent by the another device, and update the sleep-aiding audio library based on the sleep quality of the first user and the sleep quality of the second user, that is, the first device 620 and the second device 630 cooperate to perform step S530 in the method 500. For example, the first device 620 may update, based on the sleep quality of the first user, the audio signal corresponding to the first user in the sleep-aiding audio library, and the second device 630 may update, based on the sleep quality of the first user and the sleep quality of the second user, a sleep-aiding audio signal corresponding to a group user in the sleep-aiding audio library.

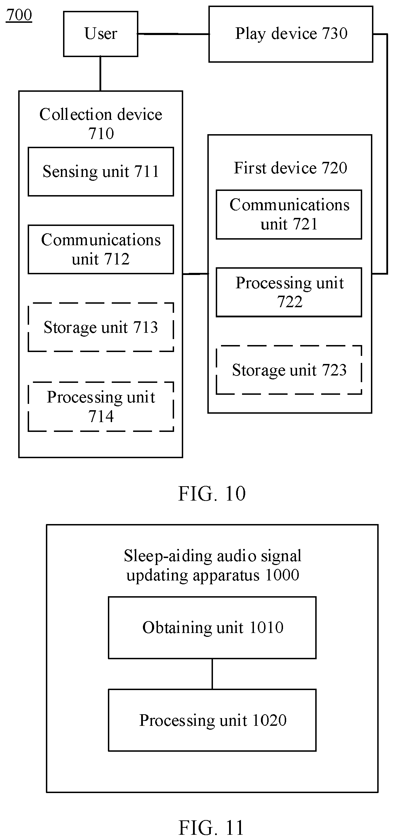

FIG. 10 shows a sleep-aiding audio signal updating system 700 according to an embodiment of this application. For example, the system 700 shown in FIG. 10 may be configured to complete functions that need to be performed by the modules in FIG. 4, or the system 700 may be configured to perform the method in FIG. 5. The system 700 may include a collection device 710, a first device 720, and a play device 730.

For example, the first device 720 may be a terminal device or a cloud server.

The collection device 710 may perform a same operation by using a same structure as the collection device 610 shown in FIG. 9, to implement a same function. For example, the collection device 710 may include a sensing unit 711 and a communications unit 712. Optionally, the collection device may further include a storage unit 713 and a processing unit 714. For detailed descriptions, refer to the foregoing collection device 610. Details are not described herein again.

The first device 720 may perform a same operation by using a same structure as the first device 620 shown in FIG. 9, to implement a same function. For example, the first device 720 may include a communications unit 721 and a processing unit 722. Optionally, the first device 720 may further include a storage unit 723. For detailed descriptions, refer to the foregoing first device 620. Details are not described herein again. It should be noted that the first device 720 may further perform an operation of the processor unit 632 in the second device 630 in FIG. 9, to implement a corresponding function.

The play device 730 may perform a same operation by using a same structure as the play device 640 shown in FIG. 9, to implement a same function. For detailed descriptions, refer to the foregoing play device 640. Details are not described herein again.

Optionally, the collection device 710 may be disposed in the first device 720. Alternatively, it may be understood that the first device 720 includes a collection unit, so that a corresponding function of the collection device 710 can be implemented. For example, the first device 720 may be a head-mounted device. For example, the head-mounted device may include a head cover, an eye cover, a headset, or a pillow.

Optionally, the play device 730 may be disposed in the first device 720. Alternatively, it may be understood that the first device 720 includes a play unit, so that a corresponding function of the play device 730 can be implemented. For example, the first device 720 may be a device such as a wearable device, a mobile terminal, or a sound box. For example, the wearable device may include a headset. For another example, the mobile terminal may include a mobile phone or a tablet computer.

That is, the collection device 710 or the play device 730 may not be an independent device, but may implement a corresponding function in a form of a unit integrated into the first device 720. Alternatively, it may be understood that the system 700 includes the first device 720. The first device 720 may be further configured to perform an operation of the collection device 710, to implement a corresponding function. Alternatively, the first device 720 may be further configured to perform an operation of the play device 730, to implement a corresponding function.

The following uses an example to describe a process of updating a sleep-aiding audio signal by using the system shown in FIG. 10.

Step 1: The first device 720 obtains a first biological signal collected when a first audio signal in a sleep-aiding audio library is played.

The first biological signal is a biological signal corresponding to a first user. Specifically, the first biological signal may be a bioelectrical signal of the user, or may be a preprocessed bioelectrical signal.

For example, the processing unit 722 may obtain the first biological signal that is sent by the collection device 710 and that is received by the communications unit 721.

For example, the first device 720 may include a collection unit, and the collection unit is configured to collect the bioelectrical signal of the user. That is, the first device 720 may not include the communications unit 721, and the processing unit 722 may obtain the bioelectrical signal collected by the collection unit.

Further, the collection unit may preprocess the bioelectrical signal, for example, filter the bioelectrical signal.

Step 2: The first device 720 determines a sleep quality of the first user based on the first biological signal. For specific descriptions, refer to step S520 in the method 500. Details are not described herein again.

Step 3: The first device 720 updates the sleep-aiding audio library based on the sleep quality of the first user. For specific descriptions, refer to step S530 in the method 500. Details are not described herein again.

Step 4: The play device 730 plays a target sleep-aiding audio signal. This step is an optional step.

For example, the target sleep-aiding audio signal may be determined based on an updated sleep-aiding audio library. For example, the target sleep-aiding audio signal may be a sleep-aiding audio signal ranked first in a plurality of sleep-aiding audio signals in the sleep-aiding audio library. For another example, a sequence of the plurality of sleep-aiding audio signals may be displayed to the first user, so that the first user can select a sleep-aiding audio signal based on the sequence.

For example, the play device 730 may obtain the updated sleep-aiding audio library, and then determine the target sleep-aiding audio signal.

For example, the play device 730 may obtain the target sleep-aiding audio signal. That is, the first device 720 may be configured to: determine the target sleep-aiding audio signal and indicate the play device 730 to play the target sleep-aiding audio signal.

For example, the first device 720 may include a play unit, and the play unit is configured to play an audio signal. That is, the first device 720 may not include the communications unit 721, and the processing unit 722 may indicate the play unit to play the target sleep-aiding audio signal.

It should be noted that the communications unit in FIG. 9 or FIG. 10 may be alternatively a transceiver circuit, an interface circuit, a transceiver, a communications module, a transceiver unit, a transceiver module, or the like, and may perform connection or communication in a wired or wireless manner, to implement communication between devices.

FIG. 11 is a schematic block diagram of a sleep-aiding audio signal updating apparatus according to an embodiment of this application. A sleep-aiding audio signal updating apparatus 1000 shown in FIG. 11 includes an obtaining unit 1010 and a processing unit 1020.

The obtaining unit 1010 and the processing unit 1020 may be configured to perform the sleep-aiding audio signal updating method in the embodiments of this application. Specifically, the processing unit 1020 may perform the foregoing method 500.

The obtaining unit 1010 is configured to obtain a first biological signal collected when a first sleep-aiding audio signal in a sleep-aiding audio library is played, where the first biological signal is a biological signal of a first user. The processing unit 1020 is configured to: determine a sleep quality of the first user based on the first biological signal; and update the sleep-aiding audio library based on the sleep quality of the first user.

Optionally, as an embodiment, the processing unit 1020 is specifically configured to: determine at least one of a plurality of sleep stages based on the first biological signal; and determine, based on the at least one sleep stage, a sleep quality corresponding to the at least one sleep stage.

Optionally, as an embodiment, the sleep-aiding audio library includes sleep-aiding audio signals corresponding to the plurality of sleep stages; and the processing unit 1020 is specifically configured to update, based on the sleep quality corresponding to the at least one sleep stage, a sleep-aiding audio signal corresponding to the at least one sleep stage in the sleep-aiding audio library, to obtain an updated sleep-aiding audio signal corresponding to the at least one sleep stage.

Optionally, as an embodiment, the processing unit 1020 is further configured to determine a target sleep-aiding audio signal based on the updated sleep-aiding audio signal corresponding to the at least one sleep stage, where the target sleep-aiding audio signal is used to be played for the first user when the first user is in the at least one sleep stage.

Optionally, as an embodiment, the processing unit 1020 is specifically configured to determine, based on duration of the at least one sleep stage and a reference value corresponding to the at least one sleep stage, the sleep quality corresponding to the at least one sleep stage.

Optionally, as an embodiment, the processing unit 1020 is further configured to: obtain feedback information of the first user for the first sleep-aiding audio signal; and update, based on the feedback information, the reference value corresponding to the at least one sleep stage.

Optionally, as an embodiment, the processing unit 1020 is specifically configured to: update a sequence of the sleep-aiding audio signals in the sleep-aiding audio library based on the sleep quality of the first user; and/or delete one or more sleep-aiding audio signals from the sleep-aiding audio library based on the sleep quality of the first user.

Optionally, as an embodiment, the first sleep-aiding audio signal is a newly added sleep-aiding audio signal.

Optionally, as an embodiment, the processing unit 1020 is further configured to determine a sleep quality of a second user, where the sleep quality of the second user is determined based on a second biological signal, the second biological signal is a biological signal of the second user, and the second biological signal is collected when a second sleep-aiding audio signal in the sleep-aiding audio library is played; and the processing unit 1020 is specifically configured to update the sleep-aiding audio library based on the sleep quality of the first user and the sleep quality of the second user.

It should be noted that the apparatus 1000 is reflected in a form of a functional unit. The term "unit" herein may be implemented in a form of software and/or hardware. This is not specifically limited.

For example, the "unit" may be a software program, a hardware circuit, or a combination thereof that implements the foregoing function. The hardware circuit may include an application-specific integrated circuit (ASIC), an electronic circuit, a processor (for example, a shared processor, a dedicated processor, or a group processor) configured to execute one or more software or firmware programs, a memory, a merged logical circuit, and/or another appropriate component that supports the described function.

Therefore, in the examples described in the embodiments of this application, the units can be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

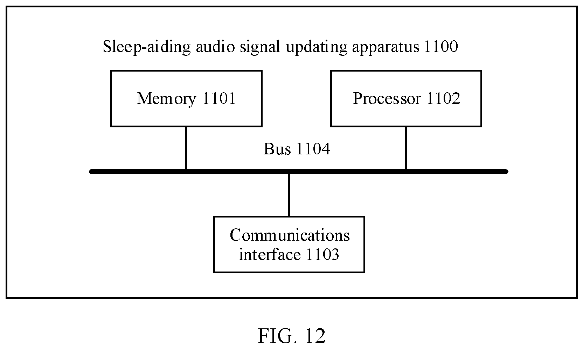

FIG. 12 is a schematic diagram of a hardware structure of a sleep-aiding audio signal updating apparatus according to an embodiment of this application. An apparatus 1100 (the apparatus 1100 may be specifically a computer device) shown in FIG. 12 includes a memory 1101, a processor 1102, a communications interface 1103, and a bus 1104. The memory 1101, the processor 1102, and the communications interface 1103 may be communicatively connected to each other by using the bus 1104.

The memory 1101 may be a read only memory (ROM), a static storage device, a dynamic storage device, or a random access memory (random access memory, RAM). The memory 1101 may store a program. When the program stored in the memory 1101 is executed by the processor 1102, the processor 1102 is configured to perform the steps of the sleep-aiding audio signal updating method in the embodiments of this application, for example, perform the steps shown in FIG. 5.

It should be understood that the apparatus shown in this embodiment of this application may be a server, for example, may be a cloud server, or may be a chip configured in the cloud server.

The processor 1102 may be a general-purpose central processing unit (CPU), a microprocessor, an application-specific integrated circuit (ASIC), a graphics processing unit (GPU), or one or more integrated circuits, and is configured to execute a related program to implement the sleep-aiding audio signal updating method in the method embodiments of this application.

The processor 1102 may be an integrated circuit chip and has a signal processing capability. In an implementation process, the steps in the method provided in this application may be implemented by using a hardware integrated logical circuit in the processor 1102, or by using instructions in a form of software.

The foregoing processor 1102 may be a general-purpose processor, a digital signal processor (DSP), an application-specific integrated circuit (ASIC), a field programmable gate array (FPGA) or another programmable logic device, a discrete gate or transistor logic device, or a discrete hardware component. The processor may implement or perform the methods, steps, and logical block diagrams that are disclosed in embodiments of this application. The general-purpose processor may be a microprocessor, or the processor may be any conventional processor or the like. The steps in the methods disclosed with reference to embodiments of this application may be directly performed and completed by a hardware decoding processor, or may be performed and completed by using a combination of hardware in the decoding processor and a software module. The software module may be located in a mature storage medium in the art, such as a random access memory, a flash memory, a read-only memory, a programmable read-only memory, an electrically erasable programmable memory, or a register. The storage medium is located in the memory 1101. The processor 1102 reads information in the memory 1101; and completes, in combination with hardware of the processor 1102, functions that need to be performed by the units included in the sleep-aiding audio signal updating apparatus shown in FIG. 11 in the embodiments of this application, or performs the method shown in FIG. 5 in the method embodiments of this application.

The communications interface 1103 uses a transceiver apparatus, including but not limited to, for example, a transceiver, to implement communication between the apparatus 1100 and another device or a communications network.

The bus 1104 may include a path for transmitting information between components (such as the memory 1101, the processor 1102, and the communications interface 1103) in the apparatus 1100.

It should be noted that although only the memory, the processor, and the communications interface in the apparatus 1100 are shown, in a specific implementation process, a person skilled in the art should understand that the apparatus 1100 may further include other components required for implementing normal running. In addition, based on a specific requirement, a person skilled in the art should understand that the apparatus 1100 may further include hardware components that implement other additional functions. In addition, a person skilled in the art should understand that the apparatus 1100 may include only components necessary for implementing the embodiments of this application, but not necessarily include all the components shown in FIG. 12.

It should also be understood that in embodiments of this application, the memory may include a read-only memory and a random access memory, and provide instructions and data to the processor. A part of the processor may further include a non-volatile random access memory. For example, the processor may further store device type information.

It should be understood that the term "and/or" in this specification describes only an association relationship between associated objects and represents that three relationships may exist. For example, A and/or B may represent the following three cases: Only A exists, both A and B exist, and only B exists. In addition, the character "/" in this specification generally indicates an "or" relationship between the associated objects.

It should be understood that sequence numbers of the foregoing processes do not mean execution sequences in embodiments of this application. The execution sequences of the processes should be determined based on functions and internal logic of the processes, and should not constitute any limitation on implementation processes of embodiments of this application.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments, and details are not described herein again.

In several embodiments provided in this application, it should be understood that the disclosed system, apparatus, and method may be implemented in another manner. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or another form.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in embodiments of this application may be integrated into one processing unit, each of the units may exist alone physically, or two or more units may be integrated into one unit.

When the functions are implemented in the form of a software functional unit and sold or used as an independent product, the functions may be stored in a computer-readable storage medium. Based on such an understanding, the technical solutions of this application essentially, or the part contributing to the conventional technology, or some of the technical solutions may be implemented in a form of a software product. The computer software product is stored in a storage medium, and includes several instructions for instructing a computer device (which may be a personal computer, a server, or a network device) to perform all or some of the steps of the methods described in embodiments of this application. The foregoing storage medium includes any medium that can store program code, such as a universal serial bus flash disk (UFD), a removable hard disk, a read-only memory (ROM), a random access memory (RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1. A sleep-aiding audio signal updating method, comprising:

   obtaining a first biological signal collected when a first sleep-aiding audio signal in a sleep-aiding audio library is played, wherein the first biological signal is a biological signal of a first user;
   determining a sleep quality of the first user based on the first biological signal; and
   updating the sleep-aiding audio library based on the sleep quality of the first user.

2. The method according to claim 1, wherein the determining a sleep quality of the first user based on the first biological signal comprises:

   determining at least one of a plurality of sleep stages based on the first biological signal; and
   determining, based on the at least one sleep stage, a sleep quality corresponding to the at least one sleep stage.

3. The method according to claim 2, wherein the sleep-aiding audio library comprises sleep-aiding audio signals corresponding to the plurality of sleep stages; and
   the updating the sleep-aiding audio library based on the sleep quality of the first user comprises:
   updating, based on the sleep quality corresponding to the at least one sleep stage, a sleep-aiding audio signal corresponding to the at least one sleep stage in the sleep-aiding audio library, to obtain an updated sleep-aiding audio signal corresponding to the at least one sleep stage.

4. The method according to claim 3, wherein the method further comprises:
   determining a target sleep-aiding audio signal based on the updated sleep-aiding audio signal corresponding to the at least one sleep stage, wherein the target sleep-aiding audio signal is used to be played for the first user when the first user is in the at least one sleep stage.

5. The method according to any one of claims 2 to 4, wherein the determining a sleep quality corresponding to the at least one sleep stage based on the at least one sleep stage comprises:

determining, based on duration of the at least one sleep stage and a reference value corresponding to the at least one sleep stage, the sleep quality corresponding to the at least one sleep stage.

6. The method according to claim 5, wherein the method further comprises:

obtaining feedback information of the first user for the first sleep-aiding audio signal; and
updating, based on the feedback information, the reference value corresponding to the at least one sleep stage.

7. The method according to any one of claims 1 to 6, wherein the updating the sleep-aiding audio library based on the sleep quality of the first user comprises:

updating a sequence of the sleep-aiding audio signals in the sleep-aiding audio library based on the sleep quality of the first user; and/or
deleting one or more sleep-aiding audio signals from the sleep-aiding audio library based on the sleep quality of the first user.

8. The method according to any one of claims 1 to 7, wherein the first sleep-aiding audio signal is a newly added sleep-aiding audio signal.

9. The method according to any one of claims 1 to 8, wherein the method further comprises:

determining a sleep quality of a second user, wherein the sleep quality of the second user is determined based on a second biological signal, the second biological signal is a biological signal of the second user, and the second biological signal is collected when a second sleep-aiding audio signal in the sleep-aiding audio library is played; and
the updating the sleep-aiding audio library based on the sleep quality of the first user comprises:
updating the sleep-aiding audio library based on the sleep quality of the first user and the sleep quality of the second user.

10. A sleep-aiding audio signal updating apparatus, comprising:

an obtaining unit, configured to obtain a first biological signal collected when a first sleep-aiding audio signal in a sleep-aiding audio library is played, wherein the first biological signal is a biological signal of a first user; and
a processing unit, configured to:

determine a sleep quality of the first user based on the first biological signal; and
update the sleep-aiding audio library based on the sleep quality of the first user.

11. The apparatus according to claim 10, wherein the processing unit is specifically configured to:

determine at least one of a plurality of sleep stages based on the first biological signal; and
determine, based on the at least one sleep stage, a sleep quality corresponding to the at least one sleep stage.

12. The apparatus according to claim 11, wherein the sleep-aiding audio library comprises sleep-aiding audio signals corresponding to the plurality of sleep stages; and
the processing unit is specifically configured to:
update, based on the sleep quality corresponding to the at least one sleep stage, a sleep-aiding audio signal corresponding to the at least one sleep stage in the sleep-aiding audio library, to obtain an updated sleep-aiding audio signal corresponding to the at least one sleep stage.

13. The apparatus according to claim 12, wherein the processing unit is further configured to:
determine a target sleep-aiding audio signal based on the updated sleep-aiding audio signal corresponding to the at least one sleep stage, wherein the target sleep-aiding audio signal is used to be played for the first user when the first user is in the at least one sleep stage.

14. The apparatus according to any one of claims 11 to 13, wherein the processing unit is specifically configured to:
determine, based on duration of the at least one sleep stage and a reference value corresponding to the at least one sleep stage, the sleep quality corresponding to the at least one sleep stage.

**15.** The apparatus according to claim 14, wherein the processing unit is further configured to:

obtain feedback information of the first user for the first sleep-aiding audio signal; and
update, based on the feedback information, the reference value corresponding to the at least one sleep stage.

**16.** The apparatus according to any one of claims 10 to 15, wherein the processing unit is specifically configured to:

update a sequence of the sleep-aiding audio signals in the sleep-aiding audio library based on the sleep quality of the first user; and/or
delete one or more sleep-aiding audio signals from the sleep-aiding audio library based on the sleep quality of the first user.

**17.** The apparatus according to any one of claims 10 to 16, wherein the first sleep-aiding audio signal is a newly added sleep-aiding audio signal.

**18.** The apparatus according to any one of claims 10 to 17, wherein the processing unit is further configured to:

determine a sleep quality of a second user, wherein the sleep quality of the second user is determined based on a second biological signal, the second biological signal is a biological signal of the second user, and the second biological signal is collected when a second sleep-aiding audio signal in the sleep-aiding audio library is played; and
the processing unit is specifically configured to:
update the sleep-aiding audio library based on the sleep quality of the first user and the sleep quality of the second user.

**19.** A sleep-aiding audio signal updating apparatus, comprising at least one processor and a memory, wherein the at least one processor is coupled to the memory, and is configured to read and execute instructions in the memory, to perform the method according to any one of claims 1 to 9.

**20.** A computer readable medium, wherein the computer readable medium stores program code, and when the computer program code is run on a computer, the computer is enabled to perform the method according to any one of claims 1 to 9.

```
                    ┌─────────────────────┐
                    │ Sleep period division │
                    └─────────────────────┘
         ┌─────────────┐  ┌──────────────┐  ┌──────────────┐
         │ Wakefulness │  │ Non-rapid eye │  │  Rapid eye   │
         └─────────────┘  │ movement sleep│  │ movement sleep│
                          │   (NREM)     │  │   (REM)      │
                          └──────────────┘  └──────────────┘
```

Sleep period division

Wakefulness

Non-rapid eye movement sleep (NREM)

Rapid eye movement sleep (REM)

Sleep period 1 (N1)

Sleep period 2 (N2)

Sleep period 3 (N3)

Sleep period 4 (N4)

Light sleep

Slow wave sleep

FIG. 1

Wakefulness (W)

Light sleep (LS)

Rapid eye
movement sleep
(REM)

Slow wave sleep
(SWS)

100

Voltage (μV)

0

0          1          2          3          4

Time (s)

FIG. 2

W

Sleep
stage

1

2

3

4

0     1     2     3     4     5     6     7     8

Sleep time (h)

FIG. 3

400

Sleep-aiding audio library updating module 470

Audio play module 420 ← Sleep-aiding audio library 410

User

Signal collection module 430

Signal analysis module 440

Audio sorting module 460

Audio evaluation module 450

FIG. 4

500

| Obtain a first biological signal collected when a first audio signal in a sleep-aiding audio library is played | S510 |
| Determine a sleep quality of a first user based on the first biological signal | S520 |
| Update the sleep-aiding audio library based on the sleep quality of the first user | S530 |
| Play a target sleep-aiding audio signal | S540 |
| Determine a sleep quality of a second user | S550 |

FIG. 5

| Sleep-aiding audio signal 1 | Sleep-aiding audio signal 2 | Sleep-aiding audio signal n |
|---|---|---|
| Sleep quality of a W period of an individual user | Sleep quality of a W period of the individual user | Sleep quality of a W period of the individual user |
| Sleep quality of an N1 period of the individual user | Sleep quality of an N1 period of the individual user | Sleep quality of an N1 period of the individual user |
| Sleep quality of an N2 period of the individual user | Sleep quality of an N2 period of the individual user | Sleep quality of an N2 period of the individual user |
| Sleep quality of an N3 period of the individual user | Sleep quality of an N3 period of the individual user | Sleep quality of an N3 period of the individual user |
| Sleep quality of an N4 period of the individual user | Sleep quality of an N4 period of the individual user | Sleep quality of an N4 period of the individual user |
| Sleep quality of a REM period of the individual user | Sleep quality of a REM period of the individual user | Sleep quality of a REM period of the individual user |
| Whole linear sleep quality of the individual user | Whole linear sleep quality of the individual user | Whole linear sleep quality of the individual user |
| Whole weighted sleep quality of the individual user | Whole weighted sleep quality of the individual user | Whole weighted sleep quality of the individual user |
| Sleep quality of a W period of a group user | Sleep quality of a W period of the group user | Sleep quality of a W period of the group user |
| Sleep quality of an N1 period of the group user | Sleep quality of an N1 period of the group user | Sleep quality of an N1 period of the group user |
| Sleep quality of an N2 period of the group user | Sleep quality of an N2 period of the group user | Sleep quality of an N2 period of the group user |
| Sleep quality of an N3 period of the group user | Sleep quality of an N3 period of the group user | Sleep quality of an N3 period of the group user |
| Sleep quality of an N4 period of the group user | Sleep quality of an N4 period of the group user | Sleep quality of an N4 period of the group user |
| Sleep quality of a REM period of the group user | Sleep quality of a REM period of the group user | Sleep quality of a REM period of the group user |
| Whole linear sleep quality of the group user | Whole linear sleep quality of the group user | Whole linear sleep quality of the group user |
| Whole weighted sleep quality of the group user | Whole weighted sleep quality of the group user | Whole weighted sleep quality of the group user |
| ... | ... | ... |

FIG. 6

| Individual user | | | |
|---|---|---|---|
| Integration result of a W period | Integration result of an N1 period | Integration result of an N2 period | Integration result of an N3 period |
| Sleep-aiding audio signal 1 Sleep quality 1 of a W period of the individual user | Sleep-aiding audio signal 1 Sleep quality 1 of an N1 period of the individual user | Sleep-aiding audio signal 1 Sleep quality 1 of an N2 period of the individual user | Sleep-aiding audio signal 1 Sleep quality 1 of an N3 period of the individual user |
| Sleep-aiding audio signal 2 Sleep quality 2 of a W period of the individual user | Sleep-aiding audio signal 2 Sleep quality 2 of an N1 period of the individual user | Sleep-aiding audio signal 2 Sleep quality 2 of an N2 period of the individual user | Sleep-aiding audio signal 2 Sleep quality 2 of an N3 period of the individual user |
| ... | ... | ... | ... |
| Sleep-aiding audio signal m Sleep quality m of a W period of the individual user | Sleep-aiding audio signal m Sleep quality m of an N1 period of the individual user | Sleep-aiding audio signal m Sleep quality m of an N2 period of the individual user | Sleep-aiding audio signal m Sleep quality 3 of an N3 period of the individual user |
| Integration result of an N4 period | Integration result of a REM period | Integration result based on a whole linear sleep quality | Integration result based on a whole weighted sleep quality |
| Sleep-aiding audio signal 1 Sleep quality 1 of an N4 period of the individual user | Sleep-aiding audio signal 1 Sleep quality 1 of a REM period of the individual user | Sleep-aiding audio signal 1 Whole linear sleep quality 1 of the individual user | Sleep-aiding audio signal 1 Whole weighted sleep quality 1 of the individual user |
| Sleep-aiding audio signal 2 Sleep quality 2 of an N4 period of the individual user | Sleep-aiding audio signal 2 Sleep quality 2 of a REM period of the individual user | Sleep-aiding audio signal 2 Whole linear sleep quality 2 of the individual user | Sleep-aiding audio signal 2 Whole weighted sleep quality 2 of the individual user |
| ... | ... | ... | ... |
| Sleep-aiding audio signal m Sleep quality m of an N4 period of the individual user | Sleep-aiding audio signal m Sleep quality m of a REM period of the individual user | Sleep-aiding audio signal m Whole linear sleep quality m of the individual user | Sleep-aiding audio signal m Whole weighted sleep quality m of the individual user |

TO
FIG. 7B

FIG. 7A

TO
FIG. 7B

Group user

| Integration result of a W period | Integration result of an N1 period | Integration result of an N2 period | Integration result of an N3 period |
|---|---|---|---|
| Sleep-aiding audio signal 1 Sleep quality 1 of a W period of the group user | Sleep-aiding audio signal 1 Sleep quality 2 of an N1 period of the group user | Sleep-aiding audio signal 1 Sleep quality 1 of an N2 period of the group user | Sleep-aiding audio signal 1 Sleep quality 1 of an N3 period of the group user |
| Sleep-aiding audio signal 2 Sleep quality 2 of a W period of the group user | Sleep-aiding audio signal 2 Sleep quality 2 of an N1 period of the group user | Sleep-aiding audio signal 2 Sleep quality 2 of an N2 period of the group user | Sleep-aiding audio signal 2 Sleep quality 2 of an N3 period of the group user |
| ... | ... | ... | ... |
| Sleep-aiding audio signal m Sleep quality m of a W period of the group user | Sleep-aiding audio signal m Sleep quality m of an N1 period of the group user | Sleep-aiding audio signal m Sleep quality m of an N2 period of the group user | Sleep-aiding audio signal m Sleep quality m of an N3 period of the group user |

| Integration result of an N4 period | Integration result of a REM period | Integration result based on a whole linear sleep quality | Integration result based on a whole weighted sleep quality |
|---|---|---|---|
| Sleep-aiding audio signal 1 Sleep quality 1 of an N4 period of the group user | Sleep-aiding audio signal 1 Sleep quality 1 of a REM period of the group user | Sleep-aiding audio signal 1 Whole linear sleep quality 1 of the individual user | Sleep-aiding audio signal 1 Whole weighted sleep quality 1 of the group user |
| Sleep-aiding audio signal 2 Sleep quality 2 of an N4 period of the group user | Sleep-aiding audio signal 2 Sleep quality 2 of a REM period of the group user | Sleep-aiding audio signal 2 Whole linear sleep quality 2 of the individual user | Sleep-aiding audio signal 2 Whole weighted sleep quality 2 of the group user |
| ... | ... | ... | ... |
| Sleep-aiding audio signal m Sleep quality m of an N4 period of the group user | Sleep-aiding audio signal m Sleep quality m of a REM period of the group user | Sleep-aiding audio signal m Whole linear sleep quality m of the group user | Sleep-aiding audio signal m Whole weighted sleep quality m of the group user |

FIG. 7B

| Individual user | | | |
|---|---|---|---|
| Sorting result corresponding to a W period | Sorting result corresponding to an N1 period | Sorting result corresponding to an N2 period | Sorting result corresponding to an N3 period |
| Sleep-aiding audio signal i Sleep quality i of a W period of the individual user | Sleep-aiding audio signal i Sleep quality i of an N1 period of the individual user | Sleep-aiding audio signal i Sleep quality i of an N2 period of the individual user | Sleep-aiding audio signal i Sleep quality i of an N3 period of the individual user |
| Sleep-aiding audio signal j Sleep quality j of a W period of the individual user | Sleep-aiding audio signal j Sleep quality j of an N1 period of the individual user | Sleep-aiding audio signal j Sleep quality j of an N2 period of the individual user | Sleep-aiding audio signal j Sleep quality j of an N3 period of the individual user |
| ... | ... | ... | ... |
| Sleep-aiding audio signal k Sleep quality k of a W period of the individual user | Sleep-aiding audio signal k Sleep quality k of an N1 period of the individual user | Sleep-aiding audio signal k Sleep quality k of an N2 period of the individual user | Sleep-aiding audio signal k Sleep quality k of an N3 period of the individual user |
| Sorting result corresponding to an N4 period | Sorting result corresponding to a REM period | Sorting result based on a whole linear sleep quality | Sorting result based on a whole weighted sleep quality |
| Sleep-aiding audio signal i Sleep quality i of an N4 period of the individual user | Sleep-aiding audio signal i Sleep quality i of a REM period of the individual user | Sleep-aiding audio signal i Whole linear sleep quality i of the individual user | Sleep-aiding audio signal i Whole weighted sleep quality i of the individual user |
| Sleep-aiding audio signal j Sleep quality j of an N4 period of the individual user | Sleep-aiding audio signal j Sleep quality j of a REM period of the individual user | Sleep-aiding audio signal j Whole linear sleep quality j of the individual user | Sleep-aiding audio signal j Whole weighted sleep quality j of the individual user |
| ... | ... | ... | ... |
| Sleep-aiding audio signal k Sleep quality k of an N4 period of the individual user | Sleep-aiding audio signal k Sleep quality k of a REM period of the individual user | Sleep-aiding audio signal k Whole linear sleep quality k of the individual user | Sleep-aiding audio signal k Whole weighted sleep quality k of the individual user |

TO
FIG. 8B

FIG. 8A

TO
FIG. 8B

Group user

| Sorting result corresponding to a W period | Sorting result corresponding to an N1 period | Sorting result corresponding to an N2 period | Sorting result corresponding to an N3 period |
|---|---|---|---|
| Sleep-aiding audio signal i Sleep quality i of a W period of the group user | Sleep-aiding audio signal i Sleep quality i of an N1 period of the group user | Sleep-aiding audio signal i Sleep quality i of an N2 period of the group user | Sleep-aiding audio signal i Sleep quality i of an N3 period of the group user |
| Sleep-aiding audio signal j Sleep quality j of a W period of the group user | Sleep-aiding audio signal j Sleep quality j of an N1 period of the group user | Sleep-aiding audio signal j Sleep quality j of an N2 period of the group user | Sleep-aiding audio signal j Sleep quality j of an N3 period of the group user |
| ... | ... | ... | ... |
| Sleep-aiding audio signal k Sleep quality k of a W period of the group user | Sleep-aiding audio signal k Sleep quality k of an N1 period of the group user | Sleep-aiding audio signal k Sleep quality k of an N2 period of the group user | Sleep-aiding audio signal k Sleep quality k of an N3 period of the group user |

| Sorting result corresponding to an N4 period | Sorting result corresponding to a REM period | Sorting result based on a whole linear sleep quality | Sorting result based on a whole weighted sleep quality |
|---|---|---|---|
| Sleep-aiding audio signal i Sleep quality i of an N4 period of the group user | Sleep-aiding audio signal i Sleep quality i of a REM period of the group user | Sleep-aiding audio signal i Whole linear sleep quality i of the individual user | Sleep-aiding audio signal i Whole weighted sleep quality i of the group user |
| Sleep-aiding audio signal j Sleep quality j of an N4 period of the group user | Sleep-aiding audio signal j Sleep quality j of a REM period of the group user | Sleep-aiding audio signal j Whole linear sleep quality j of the individual user | Sleep-aiding audio signal j Whole weighted sleep quality j of the group user |
| ... | ... | ... | ... |
| Sleep-aiding audio signal k Sleep quality k of an N4 period of the group user | Sleep-aiding audio signal k Sleep quality k of a REM period of the group user | Sleep-aiding audio signal k Whole linear sleep quality k of the group user | Sleep-aiding audio signal k Whole weighted sleep quality k of the group user |

FIG. 8B

600

| | User | | Play device 640 | |
|---|---|---|---|---|

Collection device 610
- Sensing unit 611
- Communications unit 612
- Storage unit 613
- Processing unit 614

First device 620
- Communications unit 621
- Processing unit 622
- Storage unit 623

Second device 630
- Communications unit 631
- Processing unit 632
- Storage unit 633

FIG. 9

700

User ——— Play device 730

Collection device 710

Sensing unit 711

Communications unit 712

Storage unit 713

Processing unit 714

First device 720

Communications unit 721

Processing unit 722

Storage unit 723

FIG. 10

Sleep-aiding audio signal updating apparatus 1000

Obtaining unit 1010

Processing unit 1020

FIG. 11

Sleep-aiding audio signal updating apparatus 1100

Memory 1101

Processor 1102

Bus 1104

Communications
interface 1103

FIG. 12

## INTERNATIONAL SEARCH REPORT

| International application No. |
|---|
| **PCT/CN2020/097389** |

### A. CLASSIFICATION OF SUBJECT MATTER

A61M 21/02(2006.01)i

According to International Patent Classification (IPC) or to both national classification and IPC

### B. FIELDS SEARCHED

Minimum documentation searched (classification system followed by classification symbols)

A61M21/-

Documentation searched other than minimum documentation to the extent that such documents are included in the fields searched

Electronic data base consulted during the international search (name of data base and, where practicable, search terms used)

DWPI, SIPOABS, ISI, IEEE, EI: 华为, 杨晖, 刘畅, 张慧敏, 李旭, 助眠, 助睡, 睡眠, 睡觉, 音频, 声, 音, 音乐, 乐曲, 生物信号, 脑电, 脑波, 心电, 眼电, 肌电, 更新, 库, sleep+, audio, sound, music+, EEG, electroencephalogram, ECG, EOG, EMG, updat+, database

### C. DOCUMENTS CONSIDERED TO BE RELEVANT

| Category* | Citation of document, with indication, where appropriate, of the relevant passages | Relevant to claim No. |
|---|---|---|
| X | CN 110841169 A (SHENZHEN INSTITUTES OF ADVANCED TECHNOLOGY, CHINESE ACADEMY OF SCIENCES) 28 February 2020 (2020-02-28)<br>claims 1-11, description paragraphs [0012]-[0040], figures 1-4 | 1-20 |
| A | CN 107715276 A (SHAANXI UNIVERSITY OF SCIENCE & TECHNOLOGY) 23 February 2018 (2018-02-23)<br>entire document | 1-20 |
| A | CN 110772700 A (PING AN TECHNOLOGY (SHENZHEN) CO., LTD.) 11 February 2020 (2020-02-11)<br>entire document | 1-20 |
| A | CN 107961429 A (GUANGZHOU CVTE ELECTRONIC TECHNOLOGY COMPANY LIMITED et al.) 27 April 2018 (2018-04-27)<br>entire document | 1-20 |
| A | CN 110433384 A (XILINMEN FURNITURE CO., LTD.) 12 November 2019 (2019-11-12)<br>entire document | 1-20 |
| A | JP 2013215587 A (HAMAMATSU PHOTONICS K.K.) 24 October 2013 (2013-10-24)<br>entire document | 1-20 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 February 2021** | **22 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

<table>
<tr><td colspan="3">International application No.<br>**PCT/CN2020/097389**</td></tr>
</table>

**C.     DOCUMENTS CONSIDERED TO BE RELEVANT**

| Category* | Citation of document, with indication, where appropriate, of the relevant passages | Relevant to claim No. |
|---|---|---|
| A | JP 2007195664 A (YAMAHA CORP.) 09 August 2007 (2007-08-09)<br>      entire document | 1-20 |

Form PCT/ISA/210 (second sheet) (January 2015)

INTERNATIONAL SEARCH REPORT
Information on patent family members

International application No.

**PCT/CN2020/097389**

| Patent document cited in search report | | | Publication date (day/month/year) | Patent family member(s) | | | Publication date (day/month/year) |
|---|---|---|---|---|---|---|---|
| CN | 110841169 | A | 28 February 2020 | CN | 110841169 | B | 25 September 2020 |
| CN | 107715276 | A | 23 February 2018 | None | | | |
| CN | 110772700 | A | 11 February 2020 | None | | | |
| CN | 107961429 | A | 27 April 2018 | None | | | |
| CN | 110433384 | A | 12 November 2019 | None | | | |
| JP | 2013215587 | A | 24 October 2013 | JP | 5286075 | B2 | 11 September 2013 |
| | | | | JP | 2009172371 | A | 06 August 2009 |
| | | | | JP | 5580919 | B2 | 27 August 2014 |
| JP | 2007195664 | A | 09 August 2007 | None | | | |

Form PCT/ISA/210 (patent family annex) (January 2015)